# EUROPEAN PATENT SPECIFICATION

(11) **EP 1 845 926 B1**
(45) Date of publication and mention of the grant of the patent: **02.01.2013**
(21) Application number: 06723055.7
(22) Date of filing: 01.02.2006
(51) Int. Cl.: A61J 1/00, A61L 2/00, A61M 1/02

(54) **SET OF DISPOSABLE BAGS FOR VIRAL INACTIVATION OF BIOLOGICAL FLUIDS**
EINWEGBEUTELSET ZUR VIRALEN INAKTIVIERUNG VON BIOLOGISCHEN FLÜSSIGKEITEN
ENSEMBLE DE SACS JETABLE DESTINES A L'INACATIVATION VIRALE DES FLUIDES BIOLOGIQUES

(30) Priority: 01.02.2005 EP 05290223
(43) Date of publication of application: 24.10.2007
(73) Proprietor: Research Foundation For Medical Devices, 1700 Fribourg (CH)
(72) Inventor: BURNOUF, Thierry, F-59249 Aubers (FR); EL-EKIABY, Magdy, Cairo (EG); GOUBRAN, Hadi Alphonse, 11431 Cairo (EG); RADOSEVICH, Miryana, F-59249 Aubers (FR); SOULIÉ, Valérie, 2012 Auvernier (CH)
(74) Representative: Cabinet Plasseraud
(86) International application number: PCT/EP2006/001455
(87) International publication number: WO 2006/082115

(56) References cited:
- WO-A-03/078963
- US-A- 3 872 868
- US-A- 5 744 038
- US-A- 6 099 734
- US-A1- 2001 010 802
- US-A1- 2002 094 568
- US-A1- 2004 170 649
- US-B1- 6 364 864

## Description

The present invention concerns a set system of disposable bags comprising a viral inactivation bag and a method in which the set of disposable bags is used, optionally in combination with other bags and devices for further purification and manufacture of therapeutic proteins.

The invention relates to viral inactivation of biological fluids, under aseptic conditions, such as human or animal blood plasma, blood serum and plasma fractions in the form of single units or small pools.

Human and animal blood plasma and plasma fractions play an important role in modern medicine. They are mainly used for transfusion purposes in the treatment of bleeding episodes, infectious episodes, or for prophylaxis or for pre-treating patients prior to surgery in certain clinical situations.

A major drawback in the use of human or animal blood plasma or plasma fractions is the risk of transmission of blood borne viruses, such as human immunodeficiency virus (HIV), hepatitis B virus (HBV), hepatitis C virus (HCV), and West Nile Virus

Therefore, different methods for viral inactivation of blood plasma and/or plasma fractions have been developed. For instance, the treatment with methylene blue followed by irradiation with visible light is a newly developed method for viral inactivation of blood plasma, which can be applied to single donations. Unfortunately, this method leads to some loss in protein activity, especially for factor VIII, fibrinogen and also Von Willebrand factor (VWF), and protein denaturation may be encountered at higher doses of methylene blue. In addition, issues about possible mutagenicity of methylene blue have been raised.

U.S. patent Nos. 4,481,189 and 4,540,573 describe the use of organic solvent/detergent combinations, or solvent alone to reduce by several orders of magnitude the infectivity of hepatitis viruses or other enveloped viruses contained in plasma and plasma products or added thereto.

U.S. patent No. 4,789,545 discloses a method for removal of organic solvent/detergent combinations, or solvent alone, from plasma and plasma products by partition into innocuous natural oils or synthetic triglycerides.

U.S. patent No. 5,094,960 describes the removal of solvent/detergent combinations, from plasma or plasma products by hydrophobic interaction chromatography under mild conditions, i.e. at the native concentrations of proteins, salts and other physiologic constituents.

Solvent/detergent or solvent alone treatment under appropriate conditions of reagent concentration, temperature and contact time effectively disassembles viruses that have envelope proteins associated with lipids, while having negligible effect on the molecular conformations and biological activity of sensitive plasma proteins.

However, the viral inactivation of human plasma by solvent/detergent, or solvent alone treatment has always involved the treatment of large pools of a large number of donations (100 to 500 litres, or more) and has required large and expensive pharmaceutical manufacturing facilities. Moreover, the solvent/detergent, or solvent alone, treatment is not effective against non-enveloped viruses, such as the currently known plasma-borne viruses parvovirus B19 or hepatitis A virus (HAV). Therefore, if one of the donations of the plasma pool is contaminated by a non-enveloped virus, the whole pool will be contaminated.

In addition, it has been shown, for reasons that are not clarified but could be due to the harsh industrial processing steps performed at a large scale, that the industrial solvent/detergent treatment, as applied to large pools of plasma, leads to a loss of protease inhibitor and antithrombotic/anticoagulant proteins such as Protein S and alpha-2 antiplasmin, as well as in a reduction of the high-molecular-weight multimers of Von Willebrand Factor.

One of the objectives of the present invention is to provide for a convenient, easy- to-use and cost-effective means that allows for the viral inactivation of small pools of biological fluids, such as blood plasma or plasma fractions. It is a further object to avoid protein denaturation and loss in protein activity.

The inventors have now found surprisingly and after intensive research, that the objective can be reached with a set of disposable bags as defined in claim 1 to maintain bacterial sterility during the processing step and reduce or eliminate the needs for bacterial filtration steps that induce protein losses.

Preferably, the ovoid longitudinal section of the viral inactivation bag is elliptic.

Advantageously, the elliptical longitudinal section has first horizontal axis a and a second vertical axis b, whereby the ratio a/b is greater than 1. The terms horizontal and vertical are used with respect to the position of the bag in use where the outlet facility is directed vertically downwards. The axis a corresponds to the horizontal cross-section and the axis b corresponds to the vertical cross-section.

The ovoid, preferably elliptic longitudinal section of the inner compartment has the advantage of not having any angles, which could hold back some of the biological fluid and avoid the total extensive mixing of the fluid with the viral inactivating agents.

The viral inactivation bag is made of a therapeutically acceptable and flexible material. By flexible material is understood a material that can be deformed when submitted to pressure or stress, for example due to filling and shaking the bag. The material should maintain its flexibility at temperatures in the range of 5°C to 60°C, more particularly in the range of 20 to 37°C that is commonly used to carry out viral inactivation treatment by the solvent/detergent combination or solvent alone.

A suitable material is a material which is suitable for contact with blood and plasma products and derivatives thereof, for example conventional medical/pharmaceutical grade polyvinyl chloride (PVC), as used in the blood and plasma industry.

The viral inactivation bag is preferably made of two laminated sheets of the therapeutically acceptable and flexible material, the two sheets being welded together on their periphery in order to form an ovoid inner compartment comprising at least two apertures for the inlet facilities and one for the outlet facility, respectively. The welding should resist to the pressure and stress produced by the biological fluid, especially when filling and shaking the bag. Preferably, the sheets are welded together e.g. by ultrasonic sealing or radio frequency (high energy) sealing in order to avoid contamination of the inner compartment with chemical products.

Advantageously, the viral inactivation bag comprises one at least translucent portion adjacent to the outlet facility. Preferably, the whole bag is translucent.

In a particular embodiment, the viral inactivation bag is equipped with means for suspending the bag vertically, the outlet facility showing downwards. Advantageously, the means for suspending the bag are arranged in the region of the upper periphery of the bag so that the bag can be hung up or laid without deformation. It can also be equipped with means for lying it horizontally (e.g. for the viral inactivation step under controlled temperature and mild shaking).

The volume of the inner compartment depends on the quantity of biological fluid to be treated. Advantageously, the volume of the inner compartment is in the range from 50 ml to 20 1, preferably in the range from 50 ml to 5000 ml, more preferably from 100 ml to 3000 ml, and even more preferably in the range from 200 ml to 2000 ml depending upon the plasma or plasma fraction to be virally-inactivated or the number of plasma units used as the starting pool.

The inlet facility and the outlet facility are arranged on opposite sides of the viral inactivation bag.

In a preferred embodiment, the inlet facility comprises two ports, a first port for the transfer of the biological fluid and a second port for the addition of process chemicals. By process chemical is understood any chemical product used during viral inactivation. Advantageously, the first port is provided with at least one state of the art bag spike or Luer-lock, e.g. one, or two, or three bag spikes or Luer-locks, in order to allow for an easy transfer of the biological fluid from a storage or collection bag into the viral inactivation bag.

The outlet facility allows the transfer from the viral inactivation bag to another container, for example a disposable funnel bag directly or by means of a flexible tube.

The disposable funnel bag used for the removal of the viral inactivating agent(s) comprises an inner compartment, an inlet facility and an outlet facility, which are both connected to the inner compartment, characterized in that the inner compartment has a funnel-like lower portion that meets the outlet facility and the inner compartment contains a separation agent.

Preferably the inner compartment of the funnel bag has a longitudinal section which comprises a lower portion of essentially triangular shape, the triangle being formed by the lower limitations of the longitudinal section of the inner compartment. The lower limitations are essentially linear and enclose an angle α of 15° to 170°, preferably 30° to 150° and even more preferably of 50° to 130°, e.g. 120°. This angle α coincides with the outlet facility of the funnel bag.

Preferably, the height of the triangular portion is from 10 to 100 %, preferably 15 to 50 % and even more preferably from 20 to 40 %, e.g. 25 % of the total height between the vertex of the triangle enclosing angle α and the lower end of the inlet facility of the funnel bag.

The lower portion of the inner compartment in the form of a funnel has the advantage of allowing an optimized phase separation between e.g. an upper oil phase and the biological fluid containing lower phase as well as a satisfactory, i.e. a regular and constant draining of the content and a well-controlled, potentially machine-assisted separation of biological fraction phase (bottom layer) from the oily phase (top layer).

The funnel bag is made of a therapeutically acceptable and flexible material. By flexible material is understood a material that can be deformed when submitted to pressure or stress, for example due to filling and shaking the bag. The material should maintain its flexibility at temperatures in the range of 5°C to 60°C, more particularly in the range of 20 to 37°C that is the optimal temperature for stability of protein solutions in the absence of heat-stabilizers.

A suitable material is for example conventional polyvinyl chloride (PVC) for medical/pharmaceutical use.

The funnel bag can be made of two sheets of the therapeutically acceptable and flexible material that are put congruently on top of each other and welded together on their periphery so that the inner compartment comprising two apertures for the inlet and outlet facilities, respectively, is formed. The welding should resist to the pressure and stress produced by the biological fluid, especially when filling and shaking the bag. Preferably, the sheets are welded together by ultrasonic sealing in order to avoid contamination of the inner compartment with chemical products.

Advantageously, the funnel bag comprises one at least translucent portion adjacent to the outlet facility. Preferably, the whole bag is translucent.

In a particular embodiment, the funnel bag is equipped with means for suspending the bag vertically, the outlet facility showing downwards. Advantageously, the means for suspending the bag are arranged in the region of the upper periphery of the bag so that the bag can be hung up without deformation.

The volume of the inner compartment depends essentially on the volume of the process chemicals containing biological fluid. Advantageously, the volume of the inner compartment is 10% larger than that of the viral inactivation bag, and in the range from 55 ml to 22 l, preferably in the range from, 55 ml to 5500 ml, more preferably in the range from 110 ml to 3300 ml, and even more preferably in the range from 220 ml to 2200 ml depending upon the plasma or plasma fraction to be virally-inactivated or the number of plasma units used as the starting pool.

The inlet facility and the outlet facility are preferably arranged on opposite sides of the funnel bag. Advantageously, the inlet facility comprises a state of the art plasma bag spike in order to allow for an easy transfer of the mixture into the extraction bag. For this purpose, the outlet facility of the viral inactivation bag is punctured with the spike and the mixture is transferred into the extraction bag by gravity.

Advantageously, the outlet facility of the viral inactivation bag and inlet facility of the downstream funnel bag may be connected by tubings and controlled by both breakable valves and clamps.

The outlet facility of the funnel bag is preferably sealed.

The funnel bag is adapted to contain a sterile pharmaceutical oil grade in order to perform an oil extraction followed by phase separation. In a particular embodiment, the oil is sterilized inside the funnel bag prior to the oil extraction, e.g. by autoclaving.

The pharmaceutical grade oil can be a naturally occurring oil, for example extracted from a plant or an animal, or a synthetic compound of similar structure. Suitable naturally occurring oils include castor oil (also known as ricinus oil), soybean oil, sunflower oil, cottonseed oil. A preferred synthetic compound is a synthetic triglyceride. Examples of suitable synthetic triglycerides include triolein, tristearin, tripalmitin, trimyristin, and combinations thereof.

The amount of pharmaceutical grade oil which is present in the bag is the amount that allows the extraction of at least 80% of lipid soluble process chemicals, the oil being used in an amount from 2 to 20 weight% based on the weight of the biological fluid, preferably from 5 to 15 weight% and more preferably from 5 to 10 weight%, e.g. 7.5 weight%.

The above-described funnel bag can also be used in combination with a chromatographic stationary phase. In this case, other forms of disposable bags can also be contemplated. When the stationary chromatographic phase has been treated in said disposable bag, it is transferred to a disposable column chromatography device.

Suitable stationary phases that can be used for further processing of the biological products comprise reversed-phase (hydrophobic interaction) matrices as those used for the removal of solvent or detergent viral inactivating agents, or protein adsorption matrices such as ion-exchange (anion and cation) matrices and affinity (such as immuno-affinity or immobilized heparin) matrices, or size-exclusion matrices. Preferred reversed phase matrices are a C18 silica packing material, or a SDR (solvent-detergent removal) hyper D. Preferred anion exchange matrices are, depending upon the protein to be purified, anion-exchangers gels, such as DEAE-Sephadex A-50, DEAE-Sepharose FF, Q-Sepharose, DEAE-Toyopearl 650M, DEAE-Hyper D.

For economic reasons, inexpensive stationary phases will be preferentially used in disposable bags according to the invention in order to fill disposable column chromatography bag devices. More expensive stationary phases are preferably recycled and thus used in traditional chromatographic columns which can be connected to disposable bags according to the invention, and used aseptically.

The disposable column chromatography bag device can be used more particularly for the purification of the prothrombin complex concentrate, using DEAE-Sephadex A-50.

The column chromatography bag is made of a therapeutically acceptable and semi-rigid material. By semi-rigid material it is understood a material that can be deformed when submitted to pressure or stress, for example due to filling and shaking the bag. The material should maintain its semi-rigidity at temperatures in the range of 4°C to 50°C.

In one embodiment, the set of bags according to the invention comprises two viral inactivation bags. The use of a second viral inactivation bag further increases the probability that all of the biological fluid gets into contact with the viral inactivation agent, although the use of only one bag already allows a complete viral inactivation.

In a particular embodiment, the bags of the set of disposable bags according to the invention are connected with each other, for example with flexible tubes. The tubes are advantageously equipped with means for stopping and eventually regulating the flow of biological fluid from one bag to another, such as clamps or valves.

In an embodiment for the viral inactivation of whole plasma used for transfusion, the set of disposable bags comprises at least one, preferably two viral inactivation bags according to the invention, 1 to 3, preferably 2, and more preferably 3, advantageously subsequent, funnel bags according to the invention pre-filled with sterile pharmaceutical grade oil, and a funnel bag according to the invention pre-filled with a stationary phase for column chromatography. Preferably, the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag. The outlet facility of the third funnel bag is connected to a chromatographic system. Such chromatographic system is also comprised of plastic bags containing purification buffers or solvents. If the set of disposable bags comprises two viral inactivation bags, the additional viral inactivation bag is introduced before the first viral inactivation bag, i.e. its outlet facility is connected to the inlet facility of the first viral inactivation bag.

In another embodiment for the viral inactivation of whole plasma used for transfusion, cryo-poor plasma or cryoprecipitate, the set of disposable bags comprises at least one, preferably two viral inactivation bags according to the invention, 1 to 3, preferably 2, and more preferably 3, advantageously subsequent, funnel bags according to the invention pre-filled with sterile pharmaceutical grade oil. Preferably, the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag. The outlet facility of the third funnel bag is connected to the inlet facility of a fourth bag which does not need to have funnel type shape. If the set of disposable bags comprises two viral inactivation bags, the additional viral inactivation bag is introduced before the first viral inactivation bag, i.e. its outlet facility is connected to the inlet facility of the first viral inactivation bag.

In an embodiment for the viral inactivation of cryo-poor plasma used for the purification of PCC, or any other proteins present in the cryo-poor plasma, or of cryoprecipitate, the set of disposable bags comprises at least one, preferably two viral inactivation bags according to the invention, 1 to 3, preferably 2, and more preferably 3, advantageously subsequent, funnel bags according to the invention pre-filled with pharmaceutical grade oil, and a funnel bag according to the invention pre-filled with a stationary phase for column chromatography. Preferably, the bags are connected among each other, i.e. the outlet facility of the viral inactivation bag is connected to the inlet facility of the first funnel bag, the outlet facility of the first funnel bag is connected to the inlet facility of the second funnel bag, the outlet facility of the second funnel bag to the inlet facility of the third funnel bag and the outlet facility of the third funnel bag to the inlet facility of the stationary phase containing funnel bag. The outlet facility of the stationary phase containing funnel bag is sealed. If the set of disposable bags comprises two viral inactivation bags, the additional viral inactivation bag is introduced before the first viral inactivation bag, i.e. its outlet facility is connected to the inlet facility of the first viral inactivation bag.

Notwithstanding the exact embodiment of the set of disposable bags, the bags are connected with flexible tubings. The tubings are advantageously equipped with means for stopping and eventually regulating the flow of biological fluid from one bag to another, such as clamps or valves.

Any equipment or accessories used in or with the set of disposable bags, such as tubings, clamps or valves, are made of medical and pharmaceutical acceptable material.

The set of disposable bags according to the invention is very flexible in its use. It is space saving, easily transportable and therefore can be employed, wherever viral inactivation of biological fluids may be necessary. In order to use the set of disposable bags, it is not necessary to dispose of any industrial facility such as a pharmaceutical factory. However, this single-use disposable bag system can also be used within the setting of a manufacturing facility to reduce the use of stainless steel equipment and eliminate the need for washing and sterilization during the manufacture of plasma derivatives.

Furthermore, the composition of the set of disposable bags can easily be modified. It can be used with or without a column chromatography bag device in order to adjust best to the required manufacturing process. The number of viral inactivation bags and funnel bags can also be varied. Therefore, the set of bags can be adapted to the individual needs and requirements of every case.

Another advantage of the set of bags according to the invention is that they are discarded after each use. Thus the risk of contamination of other batches is avoided.

The invention also relates to a method in which the set of disposable bags described above is used. It particularly relates to a method of viral inactivation of a biological fluid according to claim 9

Biological fluids according to the present invention comprise mammalian blood, blood plasma, blood serum, plasma fractions, precipitates from blood fractions and supernatants from blood fractionation, platelet poor plasma, cryo-poor plasma (cryosupernatant). Preferred biological fluids are blood plasma, including recovered plasma (plasma from whole blood) and apheresis plasma, cryo-poor plasma, plasma fractions, such as fractions for the purification of factor VIII, Von Willebrand factor, fibrinogen, fibronectin, prothrombin complex, Factor IX, Factor VII, Protein C, Protein S, Antithrombin, Alpha 1 antitrypsin, Cl-inhibitor, immunoglobulins, albumin, precipitates from plasma, such as cryoprecipitate, polyethylene glycol, caprylic acid, or ammonium sulphate precipitated fractions, and their corresponding supernatants, or any other precipitates or supernatants from plasma fractionation, such as cryosupernatant, supernatant I+II+III, precipitate I+II+III, supernatant II+III, precipitate II+III, supernatant I+III, precipitate II, supernatant IV, precipitate V. Plasma fractions, precipitates and supernatants may be obtained from recovered plasma (plasma from whole blood) as well as from apheresis plasma. The volume of recovered plasma from one whole blood donations is usually between 100 and 240 ml, whereas the volume of a donation of apheresis plasma is usually between 500 and 900 ml.

A preferred viral inactivation method that can be carried out in the viral inactivation bag according to the invention is the S/D (solvent/detergent) method, the solvent only method or the detergent only method, wherein the process chemicals that are added to the biological fluid are solvent/detergent combinations, solvent or combinations of solvents alone or detergent or combinations of detergents alone.

Suitable solvents are di- or trialkylphosphates, such as tri-(n-butyl)phosphate, tri-(t-butyl)phosphate, tri-(n-hexyl)phosphate, tri-(2-ethylhexyl)phosphate, tri-(n-decyl) phosphate, di-(n-butyl)phosphate, di-(t-butyl) phosphate, di-(n-hexyl)phosphate, di-(2-ethylhexyl)phosphate, di-(n-decyl)phosphate as well as dialkylphosphates with different alkyl chains. Di- or trialkylphosphates having different alkyl chains can be employed, for example ethyl di(n-butyl) phosphate. An especially preferred trialkylphosphate is tri-(n-butyl)phosphate (TnBP).

Suitable detergents include polyoxyethylene derivatives of fatty acids, partial esters of sorbitol anhydrides, for example the products commercialized under the names "Tween 80" (polyoxyethylene (20) sorbitan monooleate) also known as "polysorbate 80", "Tween 20" (polyoxyethylene (20) sorbitan monolaurate), and non-ionic oil soluble water detergents, such as oxyethylated alkylphenols sold under the names "Triton X-100" (polyoxyethylene (9-10) p-t-octyl phenol; molecular formula: t-Oct-C₆H₄-(OCH₂CH₂)ₓOH, x= 9-10) and "Triton X-45" (polyoxyethylene (4-5) p-t-octyl phenol, also called 4-(1,1,3,3-Tetramethylbutyl)phenyl-polyethylene glycol or Polyethylene glycol 4-tert-octylphenyl ether; molecular formula: t-Oct-C₆H₄-(OCH₂CH₂)ₓOH, x= ∼5).

Further contemplated detergents are sodium deoxycholate and sulfobetaines, such as N-dodecyl-N, N-dimethyl-2-ammonio-lethane sulphonate. Especially preferred detergents are "Tween 80", "Triton X-100" and "Triton X-45".

Advantageously, the solvent, if used alone, is used in an amount from 0.1 to 3 weight% with respect to the weight of the biological fluid, preferably 0.3 to 2.5% weight%, and even more preferably 2% weight%.

Advantageously, the solvent, when combined with a detergent is used in an amount from 0.1 to 2 weight% with respect to the weight of the biological fluid, preferably 0.3 to 1 % weight%, and even more preferably 1 % weight% for the viral inactivation of complex mixtures like plasma, cryo-poor plasma, or cryoprecipitate, or 0.3 weight % for the viral inactivation of more purified fractions like Factor VIII.

Advantageously, the detergent, when combined with a solvent is used in an amount from 0.1 to 2 weight% with respect to the weight of the biological fluid, preferably 0.3 to 1.5 % weight%, and even more preferably 1% % weight% for the viral inactivation of complex mixtures like plasma, cryo-poor plasma, or cryoprecipitate, or 1% weight % for the viral inactivation of more purified fractions like Factor VIII.

Advantageously, the detergent, if used alone, is used in an amount from 0.5 to 2 weight% with respect to the weight of the biological fluid, preferably 1 % weight%.

The biological fluid is treated with the solvent/detergent combination, the solvent or the detergent during a period of 4 to 8 hours, preferably 4 to 6 hours, for example 4 hours for the viral inactivation treatment of plasma, cryo-poor plasma, or cryoprecipitate using e.g. 1% TnBP and 1% Triton X-45 or Triton X-100, or 6 hours for the viral inactivation treatment of Factor VIII fraction using e.g. 0.3% TnBP and 1% Tween-80. In the case where more than one viral inactivation bag is used, the treatment period is split up between the different bags. For example, an overall treatment period of 4.5 hours may be split up between a first and a second bag as follows: 30 min in the first bag and 4 hours in the second bag. This distribution of the overall treatment period allows for good observance of good manufacturing practice, since the first bag is used for mixing the biological fluid with the process chemicals and the beginning of the viral inactivation, whereas the second bag is used for the final viral inactivation.

The solvent and/or detergent can be extracted from the biological fluid by oil extraction with pharmaceutical grade oil. For this purpose, the solvent and/or detergent containing biological fluid is preferably transferred into a funnel bag according to the present invention that holds sterile pharmaceutical grade oil.

One advantage of the funnel-like design of the lower portion of the inner compartment is the diminution of the contact surface between the oil and biological fluid phase towards the outlet facility and to a growth of the thickness of the upper phase towards the outlet facility. Therefore, the interface (zone between the 2 mobile phases) is more and more obvious towards the end of the draining of the lower phase and the two phases can be easily separated, therefore optimizing removal of the viral inactivation chemicals and plasma volume recovery.

The pharmaceutical grade oil can be a naturally occurring oil, for example extracted from a plant or an animal, or a synthetic compound of similar structure. Suitable naturally occurring oils include castor oil (also known as ricinus oil), soybean oil, sunflower oil, cottonseed oil. A preferred synthetic compound is a synthetic triglyceride. Examples of suitable synthetic triglycerides include triolein, tristearin, tripalmitin, trimyristin, and combinations thereof.

The amount of pharmaceutical grade oil which is present in the bag is the amount that allows the extraction of at least 80% of lipid soluble process chemicals, the oil being used in an amount from 2 to 20 weight% based on the weight of the biological fluid, preferably from 5 to 15 weight% and more preferably from 5 to 12 weight%, e.g. 7.5 or 10 weight%.

Oil extraction may be carried out once or more, for example 1 to 3 times, preferably twice and more preferably three times, depending in particular on the oil concentration and on the ability of further purification steps to contribute also to removal of the solvent and/or detergent.

The extraction process could work with 2 weight% oil or less, but the extraction procedure would have to be repeated more times in order to obtain a desired purity of the biological fluid, which is not preferred because it is time consuming and can cause loss of matter.

The column chromatography may be carried out subsequently to oil extraction or directly after solvent/detergent treatment.

In certain cases it is necessary to carry out an additional column chromatography step after the oil extraction(s) in order to allow for satisfactory removal of the process chemicals used for viral inactivation. An example of a detergent which can not be sufficiently removed by oil extraction and thus requires column chromatographic separation is Triton X-100.

Generally column chromatography is also used when virally inactivating Factor VIII plasma fraction in order to remove the process chemicals used for viral inactivation, purify Factor VIII and concentrate Factor VIII. Suitable types of column chromatography comprise anion-exchange immunoaffinity, affinity on immobilized Factor VIII ligands or immobilized heparin. Generally, column chromatography of virally inactivated Factor VIII is carried out directly after the viral inactivation step without any prior oil extraction.

The disposable viral inactivation bag according to the present invention can also be used for other viral inactivation methods of biological fluids using other process chemicals, for example methylene blue treatment, riboflavine (vitamin B2) treatment, acid pH treatment (generally at pH = 4, with hydrochloric acid) or caprylic acid treatment (generally at pH < 6.5).

Viral inactivating treatment of biological fluids, especially plasma, cryo-poor plasma, cryoprecipitate and other plasma fractions, such as Factor VIII plasma fraction, in a set of bags according to the invention allows for viral inactivation of small quantities of biological fluid.

This is particularly important, where the inactivation method is not effective against all types of viruses, as it is the case of the solvent/detergent treatment for non-enveloped viruses. In this way, the risk of contamination of large pools of up to several thousands litres of biological fluid is avoided.

Furthermore, in contrast to the prior art solvent/detergent (SD) viral inactivation of large pools of biological fluids, viral inactivation of single donations or small pools of biological fluids according to the invention avoids mechanical phenomena, such as aggregation, complexation, or oxidation phenomena, which all denaturize proteins.

Another advantage of the treatment of small quantities is that a tailor-made production of virally inactivated biological fluid is possible. Accordingly, a patient could for example be treated with virally inactivated plasma fractions coming exclusively from one donor, or small numbers of donors therefore decreasing the risks of exposure to any plasma-borne infectious agents.

Another advantage of the treatment as described in the present invention is the possibility to process and virally inactivate virally infected plasma, either as single donation, or as small pools, intended for therapeutic clinical studies, such as HCV positive plasma or SARS positive plasma, or any other potentially infectious plasma that contains neutralizing antibodies susceptible to treat other patients. Accordingly, a patient could for example be treated with virally inactivated HCV positive or SARS positive plasma fractions coming exclusively from one donor, or small numbers of donors therefore eliminating the risks of exposure to HCV or SARS or any plasma-borne infectious agents.

The above and other objects, features and advantages of the present invention will become more apparent from the following description, reference being made to the accompanying figures, in which
Figure 1 is a schematic longitudinal section of one embodiment of a viral inactivation bag according to the present invention;
Figure 1a is a schematic longitudinal section of another embodiment of a viral inactivation bag according to the present invention;
Figure 1b is a schematic cross sectional view along the line AA' of the embodiment of Figure 1 when the bag is empty;
Figure 1c is a schematic cross sectional view along the line AA' of the embodiment of Figure 1 when the bag is filled;
Figure 2 is a schematic longitudinal section of one embodiment of an extraction bag according to the present invention;
Figure 2a is a schematic longitudinal section of another embodiment of an extraction bag according to the present invention;
Figure 2b is a schematic cross-sectional view along the line AA' of the embodiment of Figure 2 when the bag is empty;
Figure 2c is a schematic cross-sectional view along the line AA' of the embodiment of Figure 2 when the bag is filled;
Figure 3 is a schematic longitudinal section of one embodiment of a disposable chromatographic column;
Figure 4 is a schematic longitudinal section of one embodiment of the set of disposable bags according to the present invention;
Figure 5 is a schematic longitudinal section of another embodiment of the set of disposable bags according to the present invention.

The terms horizontal, vertical, upper, and lower are used hereafter with respect to the position of the bags in use where the outlet facility is directed vertically downwards.

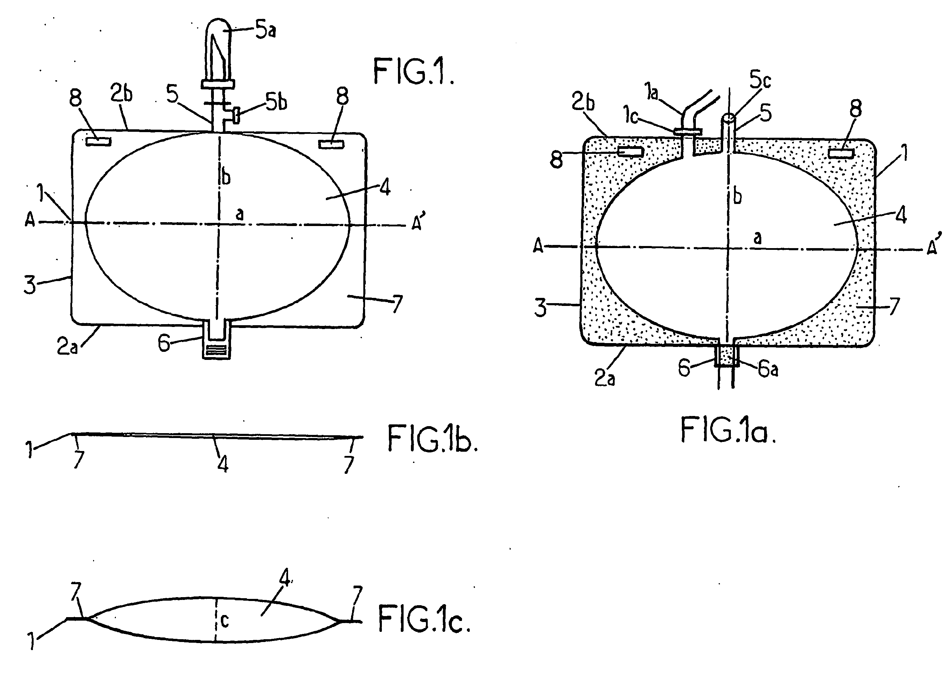

The viral inactivation bag 1 illustrated in figure 1 has an essentially rectangular outer shape, the horizontal sides 2 being longer than the vertical ones 3.

The bag 1 comprises an inner compartment 4, an inlet facility 5 and an outlet facility 6. The inner compartment 4 has a longitudinal section in the form of an ellipse with a first axis a, and a second axis b. The first axis a is parallel to the horizontal sides 2a and 2b of the bag 1, and the second axis b is parallel to its vertical sides 3, with a ratio a/b >1.

The inlet 5 and outlet 6 facilities are each separately connected to the inner compartment 4. The inlet facility 5 is arranged in the middle of the upper horizontal side 2b and coincides with the second axis b of the ellipse. The outlet facility 6 is arranged directly opposed to the inlet facility 5 in the middle of the lower horizontal side 2a and coincides as well with the second axis b of the ellipse.

The inlet facility 5 comprises a flexible tubular inlet line, a first port 5a being connected to the end of the inlet line and a second port 5b being laterally connected to the inlet line. The first port 5a is a bag spike. It is employed to transfer biological fluid from, for example, a storage or collection bag into the inner compartment 4 of the viral inactivation bag 1. The second port 5b is a port for the addition of process chemicals.

The viral inactivation bag 1 is made of two rectangular sheets of conventional medical/pharmaceutical grade polyvinylchloride (PVC) or other adequate material that are laminated and welded together on their periphery so that the inner compartment 4 comprising two apertures for the inlet 5 and outlet 6 facilities respectively is formed.

The inner compartment 4 is surrounded by a welded together border area 7 that extends to the limits of the sheets. This border area 7 comprises two holes 8, that are located left and right of the inlet facility. The holes 8 serve as means for suspending the bag 1 vertically.

Figure 1a illustrates an alternative embodiment of a viral inactivation bag 1 according to the invention. The viral inactivation bag 1 of Figure 1a, differs from the bag of Figure 1 only in that its inlet facility 5 does not comprise a first 5a and a second port 5b, but in that the bag 1 comprises a second inlet facility 1a which is arranged next to the inlet facility 5 on the upper horizontal side 2b. Generally, in this configuration, the first inlet facility 5 is used for the addition of process chemicals and the second inlet facility 1a is used for the transfer of biological fluid into the viral inactivation bag 1. The inlet facility 5 is sealed with a sealing device 5c which is suitable to be perforated, for example by a needle or canula.

The inlet facility 1a of the viral inactivation bag 1, comprises a state of the art tube clamp 1c.

The outlet facility 6 of the viral inactivation bag 1 comprises a state of the art breakable valve 6a.

Figure 1b illustrates an empty viral inactivation bag 1. In Figure 1c, the same bag is filled and the sheets are separated and bulged outwardly. The thickness c of the filled bag is smaller than the smallest of the two axis a and b.

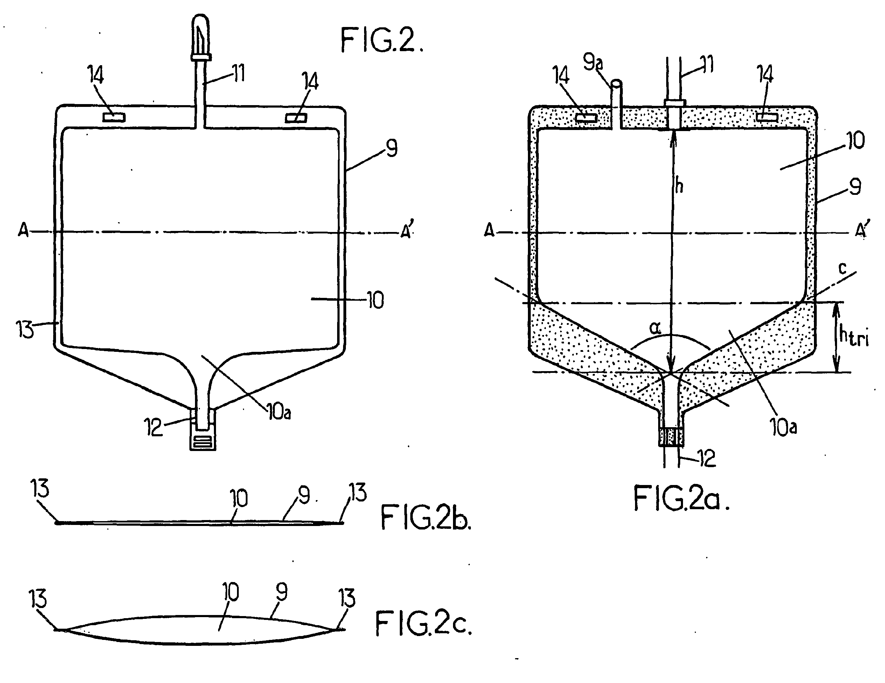

The funnel bag 9 illustrated in Figure 2 comprises an inner compartment 10, an inlet facility 11 and an outlet facility 12. The inlet 11 and outlet 12 facilities are each separately connected to the inner compartment 10 and are arranged on opposite sides of the.bag 9.

The inner compartment comprises a funnel-like lower portion 10 a.

The inlet facility 11 comprises a flexible tubular inlet line connected at one end to the inner compartment 10 and at the other end to a state of the art plasma bag spike. In this way, the process chemicals containing biological fluid can easily be transferred into the funnel bag 9 by gravity from, for example, a viral inactivation bag 1.

The funnel bag 9 is made of two sheets of conventional medical/pharmaceutical grade polyvinylchloride (PVC) or other adequate material that are laminated and welded together on their periphery so that the inner compartment 10 comprising two apertures for the inlet 11 and outlet 12 facilities respectively is formed.

The inner compartment 10 is surrounded by a welded together border area 13 that extends to the limits of the sheets. This border area 13 comprises two holes 14, that are located left and right of the inlet facility: The holes 14 serve as means for suspending the bag 9 vertically.

Figure 2a illustrates a preferred embodiment of a funnel bag 9 according to the invention.

In this embodiment, the inner compartment 10 of the funnel bag 9 has a longitudinal section which comprises a lower portion 10a of essentially triangular form, the triangle being formed by the lower limitations c and d of the longitudinal section of the inner compartment 10. The lower limitations c and d are essentially linear and enclose an angle α of about 125°. This angle α coincides with the outlet facility 12 of the funnel bag 9.

The height hₜᵣᵢ of the triangular portion is about 1/3 of the total height h between the vertex of the triangle enclosing angle α and the lower end 11a of the inlet facility 11 of the funnel bag 9.

The funnel bag 9 comprises an additional port 9a. The port is sealed with a sealing device 9c which is suitable to be perforated, for example by a needle or canula.

The inlet facility 11 of the funnel bag 9 comprises a state of the art tube clamp 11c.

The outlet facility 12 of the funnel bag 9 comprises a state of the art breakable valve 12a.

Figure 2b illustrates an empty funnel bag 9. In Figure 2c, the same bag is filled and the sheets are separated and bulged outwardly.

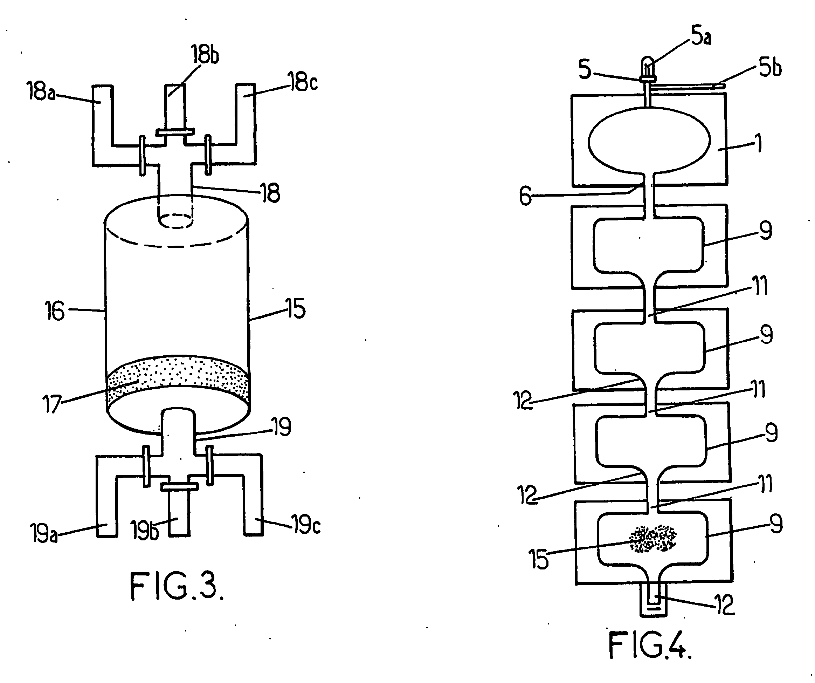

The disposable chromatographic column bag device 15 represented in Figure 3 comprises a cylindrical hollow body 16 filled with a stationary chromatographic phase, the lower part of the hollow body is provided with a filter 17. The cylindrical body is provided with an inlet facility 18 in its higher end and with an outlet facility 19 at its opposed lower end. The inlet facility comprises three ports (18a, 18b, 18c), the outlet facility also comprises three ports (19a, 19b and 19c).

The set of disposable bags illustrated in Figure 4 comprises a viral inactivation bag 1 according to the invention, three subsequent funnel bags 9 according to the invention pre-filled with pharmaceutical grade oil (the oil not being represented), and a funnel bag 9 according to the invention pre-filled with a stationary phase for column chromatography.

The bags are connected to each other with flexible tubes, i.e. the outlet facility 6 of the viral inactivation bag 1 is connected to the inlet facility 11 of the first funnel bag 9, the outlet facility 12 of the first bag 9 is connected to the inlet facility 11 of the second funnel bag 9, the outlet facility 12 of the second funnel bag 9 to the inlet facility 11 of the third funnel bag 9 and the outlet facility 12 of the third funnel bag 9 to the inlet facility 11 of the stationary phase containing funnel bag 9. The outlet facility 12 of the stationary phase containing funnel bag 9 is sealed.

The first port 5a of the inlet facility 5 of the viral inactivation bag 1 of the set of bags of Figure 4 is used for the transfer of the biological fluid into said viral inactivation bag 1. The biological fluid may directly be pooled in the first viral inactivation bag 1 by transferring it from one or more collection bags.

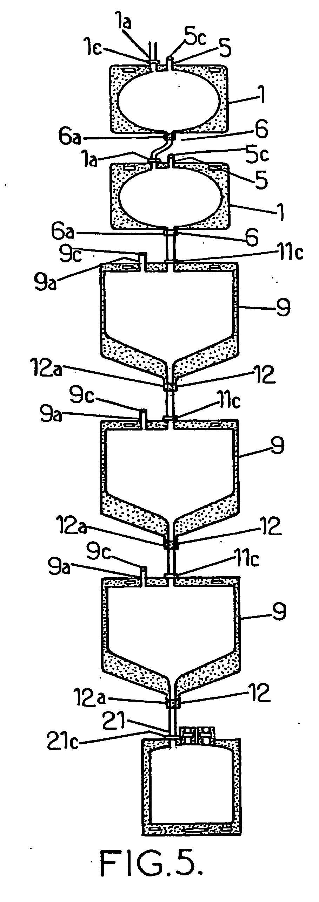

The set of disposable bags illustrated in Figure 5 comprises two viral inactivation bags 1 according to the embodiment of Figure 1a, three subsequent funnel bags 9 according to the embodiment of Figure 2a pre-filled with pharmaceutical grade oil (the oil not being represented), and a classic blood bag 20 for collecting the virally inactivated biological fluid.

The bags are connected to each other with flexible tubes, i.e. the outlet facility 6 of the first viral inactivation bag 1 is connected to the inlet facility 1a of the second viral inactivation bag 1, the outlet facility 6 of the second viral inactivation bag 1 to the inlet facility 11 of the first funnel bag 9, the outlet facility 12 of the first bag 9 is connected to the inlet facility 11 of the second funnel bag 9, the outlet facility 12 of the second funnel bag 9 to the inlet facility 11 of the third funnel bag 9 and the outlet facility 12 of the third funnel bag 9 to the inlet facility 21 of the classic blood bag 20.

The inlet facility 1a of the first viral inactivation bag 1 of the set of bags of Figure 5 is used for the transfer of the biological fluid into said viral inactivation bag 1. The biological fluid may directly be pooled in the first viral inactivation bag 1 by transferring it from one or more collection or storage bags.

The inlet facilities 1a, 11, 21 of the viral inactivation bags 1, funnel bags 9 and the classic blood bag 20 respectively comprise each a state of the art tube clamp 1c, 11c, 21c.

The outlet facilities 6, 12 of the viral inactivation bags 1 and funnel bags 9 respectively comprise each a state of the art breakable valve 6a, 12a.

In order to more fully illustrate the nature of the invention and the manner of practising the same, the following non-limiting examples are presented.

### EXAMPLES

### EXAMPLE 1: Viral inactivation of 1915 ml apheresis plasma using 2% TnBP

Apheresis plasma from three donations of about 650 ml is transferred into a sterile, single 2000 ml viral inactivation bag according to Figure 1, and the bag is heated to 31°C +/- 1°C.

A solution of pure tri(n-butyl)phosphate (TnBP) is prefilled into a sterile syringe.

Then the TnBP solution is added with the syringe (via the second port of the inactivation bag) slowly over 30 minutes to the plasma. The inactivation bag is under gentle shaking, such as using a platelet concentrate shaker device until a final concentration of 2% of the total plasma weight is reached.

After the addition of the TnBP solution, the tubing of the inlet facility is heat-sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture.

The plasma is then transferred into a funnel bag according to Figure 2 containing 100 ml of pharmaceutical grade castor oil. For this purpose, the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP with the oil.

The funnel bag is then hung up for about 30 min in order to allow for separation of a lower plasma phase and an upper TnBP containing oil phase.

The lower layer is drained by gravity through the outlet facility into a second funnel bag containing 150 ml of pharmaceutical grade castor oil.

The oil/plasma mixture in the second funnel bag is shaken vigorously until the formation of an emulsion for about one minute, then gently for 15 minutes at room temperature in order to extract further TnBP with the oil.

The second funnel bag is then hung up for about 30 min in order to allow for separation of a lower plasma phase and an upper TnBP containing oil phase.

The lower plasma layer is drained by gravity through the outlet facility into a third funnel bag containing 150 ml of pharmaceutical grade castor oil. The phase interface is detected with a UV detector that is arranged on the level of the outlet facility of the second funnel bag.

The oil/plasma mixture in the third funnel bag is shaken vigorously until the formation of an emulsion for about one minute, then gently for 15 minutes at room temperature in order to extract further TnBP with the oil.

The third funnel bag is then hung up for about 30 min in order to allow for separation of a lower plasma phase and an upper TnBP containing oil phase.

Finally, the lower plasma layer is drained by gravity through the outlet facility into a classical plasma storage bag.

Factor VIII activity, Factor IX activity, and global coagulation tests PT (Prothrombin time) and APTT (activated partial thromboplastin time) are performed in the plasma after the various steps:

| | Plasma | Plasma + 2% TnBP | 1 oil | 2 oil | 3 oil |
|---|---|---|---|---|---|
| PT, sec | 15.1 | 20.7 | 16.7 | 14.7 | 14.3 |
| PTT, sec | 41.9 | 66.7 | 40.9 | 41.1 | 43.1 |
| FVIII, % | 85 | 67 | 54 | 72 | 86 |
| FIX, % | 75 | 53 | 90 | 86 | 88 |

The results show that global coagulant activity of plasma as assessed by PT and PTT is preserved when comparing the plasma after 3 oil extractions to the starting plasma. Similarly, the recovery of FVIII and FIX is excellent.

Data also show that the PT and PTT are prolonged when plasma contains 2% TnBP and normalized after 2 oil extractions, illustrating the removal of the TnBP.

### EXAMPLE 2: Viral inactivation of 200 ml recovered plasma with 2% TnBP at 31°C

Recovered plasma from one donation of about 200 ml is transferred into a sterile, single 200 ml viral inactivation bag according to Figure 1, and the bag is heated to 31°C +/- 1°C.

A solution of pure tri(n-butyl)phosphate (TnBP) is prefilled into a sterile syringe.

Then, the TnBP solution is added with the syringe (via the second port of the inactivation bag) slowly over 30 minutes to the plasma. The inactivation bag is under gentle shaking, such as using a platelet concentrate shaker device until a final concentration of 2% of the total plasma weight is reached.

After the addition of the TnBP solution the tubing of the inlet facility is heat-sealed in order to isolate the inactivation bag.

Then vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture

The plasma is then transferred into a funnel bag according to Figure 2 containing 10 ml pharmaceutical grade castor oil. For this purpose, the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP using the oil.

The funnel bag is then hung up in order to allow for separation of a lower plasma phase and an upper TnBP containing oil phase.

The lower layer is drained by gravity through the outlet facility into a second funnel bag containing 10 ml of pharmaceutical grade castor oil.

The oil/plasma mixture in the second funnel bag is shaken vigorously until the formation of an emulsion for about one minute, then gently for 15 minutes at room temperature in order to extract further TnBP with the oil.

The second funnel bag is then hung up in order to allow for separation of a lower plasma phase and an upper TnBP containing oil phase.

The lower plasma layer is drained by gravity through the outlet facility into a third funnel bag containing 10 ml of pharmaceutical grade castor oil. The phase interface is detected with the naked eye.

The oil/plasma mixture in the third funnel bag is shaken vigorously until the formation of an emulsion for about one minute, then gently for 15 minutes at room temperature in order to extract further TnBP with the oil.

The third funnel bag is then hung up in order to allow for separation of a lower plasma phase and an upper TnBP containing oil phase.

Finally, the lower plasma layer is drained by gravity through the outlet facility into a classical plasma storage bag.

The TnBP is measured in the plasma after each extraction step:

| **Step** | **TnBP content measured by gas chromatography** |
|---|---|
| Plasma with 2% TnBP | 2% |
| Plasma after 1 oil extraction | 1.2% |
| Plasma after 2 oil extractions | 320 ppm |
| Plasma after 3 oil extractions | < 10 ppm |

Results show that TnBP is not detectable (< 10 ppm) in the final plasma after 3 oil extractions.

The PT, APTT, INR (international normalized ratio), and FVIII and FIX physiological functions are also determined at all steps:

| | Plasma | SD | 1 oil | 2 oil | 3 oil |
|---|---|---|---|---|---|
| PT, sec | 13.7 | 20.9 | 16.1 | 14.7 | 14.7 |
| INR | 1.31 | 2.68 | 1.72 | 1.47 | 1.47 |
| APTT, sec | 40.1 | 74.4 | 41.9 | 41.9 | 40.7 |
| FVIII, % | 70.7 | 89.9 | 84.9 | 70.7 | 81 |
| FIX, % | 83.3 | 81 | 130 | 87.3 | 76.5 |

Results show the good recovery in coagulant activity achieved as well as the normalisation of the value of all assays after 3 oil extractions.

### EXAMPLE 3: Recovery of protein C, Protein S, alpha 2 antiplasmin, and Von Willebrand factor ristocetin cofactor activity in 200 ml apheresis plasma virally inactivated with 2% TnBP

Apheresis plasma from one donation of about 200 ml is transferred into a sterile, single 200 ml viral inactivation bag according to Figure 1 and the bag is heated to 31°C +/- 1°C.

A solution of pure tri(n-butyl)phosphate (TnBP) is prefilled into a sterile syringe.

Then, the TnBP solution is added with the syringe (via the second port of the inactivation bag) slowly over 30 minutes to the plasma. The viral inactivation bag is under gentle shaking, such as using a platelet concentrate shaker device of the bag until a final concentration of 2% of the total plasma weight is reached.

After the addition of the TnBP solution, the tubing of the inlet facility is heat sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture.

The plasma is then transferred into a funnel bag according to Figure 2 containing 10 ml of pharmaceutical grade castor oil. For this purpose, the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, then gently for 15 minutes at room temperature in order to extract TnBP using the oil.

The funnel bag is then hung up for about 30 min in order to allow for separation of a lower plasma phase and an upper TnBP containing oil phase.

The lower layer is drained by gravity through the outlet facility into a second funnel bag containing 10 ml of pharmaceutical grade castor oil.

The oil/plasma mixture in the second funnel bag is shaken vigorously until the formation of an emulsion for about one minute, then gently for 15 minutes at room temperature in order to extract further TnBP with the oil.

The second funnel bag is then hung up in order to allow for separation of a lower plasma phase and an upper TnBP containing oil phase.

The lower plasma layer is drained by gravity through the outlet facility into a third funnel bag containing 10 ml of pharmaceutical grade castor oil. The phase interface is detected with the naked eye.

The oil/plasma mixture in the third funnel bag is shaken vigorously until the formation of an emulsion for about one minute, then gently for 15 minutes at room temperature in order to extract further TnBP with the oil.

The third funnel bag is then hung up in order to allow for separation of a lower plasma phase and an upper TnBP containing oil phase.

Finally, the lower plasma layer is drained by gravity through the outlet facility into a classical plasma storage bag.

The values of Protein C, Protein S, alpha 2 antiplasmin, and Von Willebrand factor ristocetin cofactor activity are determined at all steps:

| | Plasma | SD | 1 oil | 2 oil | 3 oil |
|---|---|---|---|---|---|
| Protein C, % | 74.1 | 59.4 | 66.2 | 66.7 | 60.8 |
| Protein S % | 70.6 | 22.8 | 61.8 | 67.7 | 69.5 |
| Alpha 2 antiplasmin % | 122.9 | 178.7 | 133.7 | 136.6 | 140.6 |
| Von Willebrand Factor, Ristocetin cofactor, % | 84 | 84 | 112 | 84 | 84 |

The results show the good recovery in the physiological functions achieved after 3 oil extractions.

### EXAMPLE 4: Viral inactivation of 200 ml recovered plasma with 1% TnBP and 1% Triton X-45

Recovered plasma from one donation of about 200 ml is transferred into a sterile, single 200 ml viral inactivation bag according to Figure 1 and the bag is heated to 31°C +/- 1°C.

A 1:1 solution of pure tri(n-butyl)phosphate (TnBP) and Triton X-45 is pre-filled into a sterile syringe.

Then, the TnBP/Triton X-45 solution is added with the syringe (via the second port of the inactivation bag) slowly over 30 minutes to the plasma. The inactivation bag is under gentle shaking, such as using a platelet concentrate shaker device until a final concentration of 1% TnBP and 1% Triton X-45 of the total plasma weight is reached.

After the addition of the TnBP/Triton X-45 solution, the tubing of the inlet facility is heat-sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture

The plasma is then transferred into a funnel bag according to Figure 2 containing 15 ml (i.e. 7.5 wt% with respect to the weight of the plasma) of pharmaceutical grade castor oil. For this purpose, the outlet facility of the viral inactivation bag is pierced with the spike of the inlet facility of the funnel bag and the TnBP and Triton X-45 containing plasma is transferred by gravity.

The oil/plasma mixture is shaken vigorously until the formation of an emulsion for about one minute, then gently for 15 minutes at room temperature in order to extract TnBP and Triton X-45 using the oil.

The funnel bag is then hung up in order to allow for separation of a lower plasma phase and an upper TnBP and Triton X-45 containing oil phase.

The lower layer is drained by gravity through the outlet facility into a second funnel bag containing 15 ml of pharmaceutical grade castor oil. The phase interface is detected with the naked eye.

The oil/plasma mixture in the second funnel bag is shaken vigorously until the formation of an emulsion for about one minute, then gently for 15 minutes at room temperature in order to extract further TnBP and Triton X-45 with the oil.

The second funnel bag is then hung up in order to allow for separation of a lower plasma phase and an upper TnBP and Triton X-45 containing oil phase.

The lower plasma layer is drained by gravity through the outlet facility into a third funnel bag containing 15 ml of pharmaceutical grade castor oil.

The oil/plasma mixture in the third funnel bag is shaken vigorously until the formation of an emulsion for about one minute, then gently for 15 minutes at room temperature in order to extract still further TnBP and Triton X-45 with the oil.

The third funnel bag is then hung up in order to allow for separation of a lower plasma phase and an upper TnBP and Triton X-45 containing oil phase.

Finally, the lower plasma layer is drained by gravity through the outlet facility into a classical plasma storage bag.

The TnBP and Triton X-45 contents are measured in the plasma after each extraction step:

| **Step** | **TnBP content measured by gas chromatography** | **Triton X-45 content measured by HPLC** |
|---|---|---|
| Plasma with 1% TnBP & 1% Triton X-45 | 1% | 1% |
| Plasma after 1 oil extraction | 550 ppm | 0.3% |
| Plasma after 2 oil extractions | < 10 ppm | 250 ppm |
| Plasma after 3 oil extractions | < 10 ppm | < 10 ppm |

The results show that TnBP and Triton X-45 are not detectable (< 10 ppm) in the final plasma after 2 or 3 oil extractions, respectively.

The PT, PTT, INR (international normalized ratio),and FVIII and FIX physiological functions are also determined at all steps:

| | Plasma | SD | 1 oil | 2 oil | 3 oil |
|---|---|---|---|---|---|
| PT, sec | 12.39 | 46.92 | 26.97 | 14.2 | 13.40 |
| INR | 1.07 | 11.39 | 4.70 | 1.29 | 1.25 |
| APTT, sec | 30.74 | > 200 | 39.67 | 32.5 | 31.78 |
| FVIII, % | 131 | 125 | 126 | 135 | 136 |
| FIX, % | 102 | 74 | 79 | 92 | 98 |

The results show the good recovery in coagulant activity achieved as well as the normalisation of the value of all assays after 3 oil extractions.

### EXAMPLE 5: Viral inactivation of 200 ml recovered plasma with 1% TnBP and 1% Triton X-100

Recovered plasma from one donation of about 200 ml is transferred into a sterile, single 200 ml viral inactivation bag according to Figure 1 and the bag is heated to 31°C +/- 1°C.

A 1:1 solution of pure tri(n-butyl)phosphate (TnBP) and Triton X-100 is pre-filled into a sterile syringe.

Then, the TnBP/Triton X-100 solution is added with the syringe (via the second port of the inactivation bag) slowly over 30 minutes to the plasma. The inactivation bag is under gentle shaking, such as using a platelet concentrate shaker device until a final concentration of 1% TnBP and 1% Triton X-100 of the total plasma weight is reached.

After the addition of the TnBP/Triton X-100 solution, the tubing of the inlet facility is heat-sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the plasma/SD mixture

The plasma is then transferred into a funnel bag according to Figure 2 containing 15 ml (i.e. 7.5 wt% with respect to the weight of the plasma) of pharmaceutical grade castor oil. For this purpose, the outlet facility of the viral inactivation bag is pierced with the spike of the inlet facility of the funnel bag and the TnBP and Triton X-100 containing plasma is transferred by gravity.

The oil/plasma mixture is shaken vigorously until the formation of an emulsion for about one minute, then gently for 15 minutes at room temperature in order to extract TnBP and minor amounts of Triton X-100 using the oil.

The funnel bag is then hung up in order to allow for separation of a lower plasma phase and an upper oil phase containing TnBP and minor amounts of Triton X-100.

The lower layer is drained by gravity through the outlet facility into a second funnel bag containing 15 ml of pharmaceutical grade castor oil. The phase interface is detected with the naked eye.

The oil/plasma mixture in the second funnel bag is shaken vigorously until the formation of an emulsion for about one minute, then gently for 15 minutes at room temperature in order to extract further TnBP and minor amounts of Triton X-100 with the oil.

The second funnel bag is then hung up in order to allow for separation of a lower plasma phase and an upper oil phase containing TnBP and minor amounts of Triton X-100.

The lower plasma layer is drained by gravity through the outlet facility into a third funnel bag containing 15 ml of pharmaceutical grade castor oil.

The oil/plasma mixture in the third funnel bag is shaken vigorously until the formation of an emulsion for about one minute, then gently for 15 minutes at room temperature in order to extract still further TnBP and minor amounts of Triton X-100 with the oil.

The third funnel bag is then hung up in order to allow for separation of a lower plasma phase and an upper oil phase containing TnBP and minor amounts of Triton X-100. Subsequent to the third oil extraction, the plasma is transferred to a centrifugation bag and centrifuged in a blood bank centrifuge at 3,900 rpm for 30 min at 20 °C.

Then, the plasma is subjected to column chromatography on a C18 chromatographic column to remove the major amount of Triton X-100.

The TnBP and Triton X-100 contents are measured in the plasma after each extraction step and after column chromatography:

| **Step** | **TnBP content measured by gas chromatography** | **Triton X-100 content measured by HPLC** |
|---|---|---|
| Plasma with 1% TnBP & 1% Triton X-100 | 1% | 1% |
| Plasma after 1 oil extraction | 520 ppm | 0.87% |
| Plasma after 2 oil extractions | 54 ppm | 0.82% |
| Plasma after 3 oil extractions | < 10 ppm | 0.78% |
| Plasma after column chromatography | < 10 ppm | < 10 ppm |

The results show that TnBP and Triton X-100 are not detectable (< 10 ppm) in the final plasma after 3 oil extractions, centrifugation and C18 chromatography.

The PT, PTT, INR (international normalized ratio), and FVIII and FIX physiological functions are also determined at all steps:

| | Plasma | SD | 1 oil | 2 oil | 3 oil | Centrifugation | Chromatography |
|---|---|---|---|---|---|---|---|
| PT, sec | 13.8 | 17.1 | 45.7 | 33.9 | 30.9 | 30.8 | 13.9 |
| INR | 1.15 | 1.68 | 9.52 | 5.62 | 4.78 | 4.75 | 1.12 |
| PTT, sec | 31 | >200 | 86.2 | 59.2 | 56.8 | 58.7 | 35 |
| FVIII, % | 132.4 | 117.5 | 112.2 | 77.6 | 64.2 | 75.2 | 125.9 |
| FIX, % | 117.6 | 54.7 | 57.3 | 43.7 | 71.3 | 85.7 | 97.8 |

The results show the good recovery in coagulant activity achieved as well as the normalisation of the value of all assays after 3 oil extractions.

### Example 6: Viral inactivation of 200 ml recovered plasma with 2% TnBP at 37°C

Recovered plasma from one donation of about 200 ml is transferred into a sterile, single 200 ml viral inactivation bag according to Figure 1 and the bag is heated to 37°C +/- 1°C.

A solution of pure tri(n-butyl)phosphate (TnBP) is prefilled into a sterile syringe.

Then, the TnBP solution is added with the syringe (via the second port of the inactivation bag) slowly over 30 minutes to the plasma. The inactivation bag is under gentle shaking, such as using a platelet concentrate shaker device until a final concentration of 2% of the total plasma weight is reached.

After the addition of the TnBP solution, the tubing of the inlet facility is heat-sealed in order to isolate the inactivation bag.

Then, vigorous mixing is performed for 30 sec, and the bag is maintained at 37°C +/- 1°C for 4 hrs under gentle stirring such as using a platelet concentrate shaker device or any other appropriate device ensuring mild constant mixing of the plasma/SD mixture.

The plasma is then transferred into a funnel bag according to Figure 2 containing 10 ml of pharmaceutical grade castor oil. For this purpose the outlet facility of the viral inactivation bag is pierced with the spike of the inlet facility of the funnel bag and the TnBP containing plasma is transferred by gravity.

The oil/plasma mixture is shaken vigorously until the formation of an emulsion for about one minute, the gently for 15 minutes at room temperature in order to extract TnBP using the oil.

The funnel bag is then hung up for about 30 min in order to allow for separation of a lower plasma phase and an upper TnBP containing oil phase.

The lower layer is drained by gravity through the outlet facility into a second funnel bag containing 15 ml of pharmaceutical grade castor oil. The phase interface is detected with a UV detector that is arranged on the level of the outlet facility of the first funnel bag.

The oil/plasma mixture in the second funnel bag is shaken vigorously until the formation of an emulsion for about one minute, then gently for 15 minutes at room temperature in order to extract further TnBP with the oil.

The second funnel bag is then hung up in order to allow for separation of a lower plasma phase and an upper TnBP containing oil phase.

The lower plasma layer is drained by gravity through the outlet facility into a third funnel bag containing 15 ml of pharmaceutical grade castor oil.

The oil/plasma mixture in the third funnel bag is shaken vigorously until the formation of an emulsion for about one minute, then gently for 15 minutes at room temperature in order to extract further TnBP with the oil.

The third funnel bag is then hung up in order to allow for separation of a lower plasma phase and an upper TnBP containing oil phase.

Subsequent to the third oil extraction, the plasma is subjected to clarifying centrifugation at 3,700 rpm for 30 min at 20°C.

The TnBP is measured in the plasma after each extraction step:

| **Step** | **TNBP content measured by gas chromatography** |
|---|---|
| Plasma with 2% TnBP | 2% |
| Plasma after 1 oil extraction | 0.9% |
| Plasma after 2 oil extractions | 85 ppm |
| Plasma after 3 oil extractions | < 10 ppm |

The results show that TnBP is not detectable (< 10 ppm) in the final plasma after 3 oil extractions.

The PT, APTT, INR (international normalized ratio), and FVIII and FIX physiological functions are also determined at all steps

| | Plasma | SD | 1 oil | 2 oil | 3 oil | Centrifugation |
|---|---|---|---|---|---|---|
| PT, sec | 15,1 | 20,7 | 16,7 | 14,7 | 14,5 | 14,3 |
| INR | 1.15 | 2.90 | 1.80 | 1.35 | 1,20 | 1.18 |
| APTT, sec | 35,9 | 66,7 | 40,9 | 41,1 | 39.5 | 37.5 |
| FVIII, % | 89 | 65 | 65 | 77 | 85 | 88 |
| FIX, % | 84 | 56 | 75 | 86 | 90 | 87 |

The results show the good recovery in coagulant activity achieved as well as the normalisation of the value of all assays after 3 oil extractions.

### EXAMPLE 7: Viral inactivation of 200 ml cryo-poor plasma with 1% TnBP and 1% Triton X-45

200 ml cryo-poor plasma were treated with 1% TnBP and 1% Triton X-45 according to the protocol of Example 4.

The TnBP and Triton X-45 contents are measured in the Cryo-poor plasma after each extraction step:

| **Step** | **TnBP content measured by gas chromatography** | **Triton X-45 content measured by HPLC** |
|---|---|---|
| Cryo-poor Plasma with 1% TnBP & 1% Triton X-45 | 1% | 1% |
| Cryo-poor Plasma after 1 oil extraction | 0.57% | 0.4% |
| Cryo-poor Plasma after 2 oil extractions | 45ppm | 240ppm |
| Cryo-poor Plasma after 3 oil extractions | Undetectable (< 10 ppm) | Undetectable (< 10 ppm) |

The results show that TnBP and Triton X-45 are not detectable (< 10.ppm) in the final Cryo-poor plasma after 3 oil extractions.

The PT, APTT, INR (international normalized ratio), FVII and FIX are also determined at all steps:

| | Cryo-poor Plasma | SD | 1 oil | 2 oil | 3 oil |
|---|---|---|---|---|---|
| PT, sec | 14.6 | 55.9 | 23.8 | 14.8 | 15.0 |
| INR | 1.6 | 12.5 | 4.89 | 1.90 | 1.76 |
| APTT, sec | 40.5 | 62.9 | 56.6 | 44.8 | 42.5 |
| FIX, % | 95 | 75 | 88 | 85 | 93 |

The results show the good recovery in coagulant activity achieved as well as the normalisation of the value of all assays after 3 oil extractions. The longer clotting times of PT and APTT compared to that of plasma are normal since cryo-poor plasma is depleted in some coagulation factors.

### EXAMPLE 8 : Viral inactivation of 200 ml cryo-poor plasma with 1% TnBP and 1% Triton X-100.

200 ml cryo-poor plasma were treated with 1% TnBP and 1% Triton X-100 according to the protocol of Example 5.

Cryo-poor plasma obtained from one donation of about 200 ml recovered plasma is transferred into a sterile, single 200 ml viral inactivation bag according to Figure 1 and the bag is heated to 31°C +/- 1°C.

A 1:1 solution of pure tri(n-butyl)phosphate (TnBP) and Triton X-100 is pre-filled into a sterile syringe.

Then, the TnBP/Triton X-100 solution is added with the syringe (via the second port of the inactivation bag) slowly over 30 minutes to the cryo-poor plasma. The inactivation bag is under gentle shaking, such as using a platelet concentrate shaker device until a final concentration of 1% TnBP and 1% Triton X-100 of the total cryo-poor plasma weight is reached.

After the addition of the TnBP/Triton X-100 solution, the tubing of the inlet facility is heat-sealed in order to isolate the inactivation bag.

Then, vigorous mixing is performed for 30 sec, and the bag is maintained at 31°C +/- 1°C for 4 hrs under gentle stirring such as using a platelet concentrate shaker device or any other appropriate device ensuring mild constant mixing of the cryo-poor plasma/SD mixture

The cryo-poor plasma is then transferred into a funnel bag according to Figure 2 containing 15 ml (i.e. 7.5 wt% with respect to the weight of the cryo-poor plasma) of pharmaceutical grade castor oil. For this purpose, the outlet facility of the viral inactivation bag is pierced with the spike of the inlet facility of the funnel bag and the TnBP and Triton X-100 containing cryo-poor plasma is transferred by gravity.

The oil/cryo-poor plasma mixture is shaken vigorously until the formation of an emulsion for about one minute, then gently for 15 minutes at room temperature in order to extract TnBP and minor amounts of Triton X-100 using the oil.

The funnel bag is then hung up in order to allow for separation of a lower cryo-poor plasma phase and an upper oil phase containing TnBP and minor amounts of Triton X-100.

The lower layer is drained by gravity through the outlet facility into a second funnel bag containing 15 ml of pharmaceutical grade castor oil. The phase interface is detected with the naked eye.

The oil/cryo-poor plasma mixture in the second funnel bag is shaken vigorously until the formation of an emulsion for about one minute, then gently for 15 minutes at room temperature in order to extract further TnBP and minor amounts of Triton X-100 with the oil.

The second funnel bag is then hung up in order to allow for separation of a lower cryo-poor plasma phase and an upper oil phase containing TnBP and minor amounts of Triton X-100.

The lower cryo-poor plasma layer is drained by gravity through the outlet facility into a third funnel bag containing 15 ml of pharmaceutical grade castor oil.

The oil/cryo-poor plasma mixture in the third funnel bag is shaken vigorously until the formation of an emulsion for about one minute, then gently for 15 minutes at room temperature in order to extract still further TnBP and minor amounts of Triton X-100 with the oil.

The third funnel bag is then hung up in order to allow for separation of a lower plasma phase and an upper oil phase containing TnBP and minor amounts of Triton X-100.

Subsequent to the third oil extraction, the plasma is transferred to a centrifugation bag and centrifuged in a blood bank centrifuge at 3,900 rpm for 30 min at 20 °C.

Then, the plasma is subjected to column chromatography on a SDR chromatographic column to remove the major amount of Triton X-100.

The TnBP and Triton X-100 contents are measured in the plasma after each extraction step and after column chromatography:
The TnBP and Triton X-100 contents are measured in the Cryo-poor plasma after each extraction step and after column chromatography on SDR:

| **Step** | **TnBP content measured by gas chromatography** | **Triton X-100 content measured by HPLC** |
|---|---|---|
| Cryo-poor Plasma with 1% TnBP & 1% Triton X-100 | 1% | 1% |
| Cryo-poor Plasma after 1 oil extraction | 0.45 % | 0.85% |
| Cryo-poor Plasma after 2 oil extractions | 62ppm | 0.78% |
| Cryo-poor Plasma after 3 oil extractions | Undetectable (< 10 ppm) | 0.70% |
| Cryo-poor Plasma after column chromatography | Undetectable (< 10 ppm) | Undetectable (< 10 ppm) |

The results show that TnBP and Triton X-100 are not detectable (< 10 ppm) in the final Cryo-poor plasma after 3 oil extractions and column chromatography, respectively.

The PT, APTT, INR (international normalized ratio), FVII and FIX are also determined at all steps:

| | Plasma | SD | 1 oil | 2 oil | 3 oil | Centrifugation | SDR Chromatography |
|---|---|---|---|---|---|---|---|
| PT, sec | 14.2 | >200 | 43.1 | 35.7 | 33.8 | 27.7 | 13.1 |
| INR | 1.21 | >200 | 8.58 | 5.87 | 4.23 | 3.94 | 1.05 |
| APTT, sec | 37.8 | >200 | >200 | 156 | 87 | 57.6 | 64.2 |
| FVII, % | 65.3 | 21.3 | 16.6 | 38.8 | 54.9 | 46.5 | 74.4 |
| FIX, % | 77.7 | 13.6 | 80.,7 | 78.9 | 80.8 | 69.5 | 88.8 |

The results show the good recovery in coagulant activity achieved (Factor VII and Factor IX) as well as the normalisation of the value of all assays after the SDR chromatographic step.

### EXAMPLE 9: Viral inactivation of 200 ml cryo-poor plasma with 2% TnBP at 37°C

200 ml cryo-poor plasma were treated with 2% TnBP according to the protocol of Example 6.

The TnBP is measured in the Cryo-poor plasma after each extraction step:

| **Step** | **TnBP content measured by gas chromatography** |
|---|---|
| Cryo-poor Plasma with 2% TnBP | 2% |
| Cryo-poor Plasma after 1 oil extraction | 350 ppm |
| Cryo-poor Plasma after 2 oil extractions | 90 ppm |
| Cryo-poor Plasma after 3 oil extractions | Undetectable (< 10 ppm) |

The results show that TnBP is not detectable (< 10 ppm) in the final Cryo-poor plasma after 3 oil extractions.

The PT, PTT, INR (international normalized ratio), and FVIII and FIX physiological functions are also determined at all steps.

| | Plasma | SD | 1 oil | 2 oil | 3 oil | Centrifugation |
|---|---|---|---|---|---|---|
| PT, sec | 14.2 | 21.2 | 20.6 | 17.6 | 15.2 | 15 |
| INR | 1.21 | 2.46 | 2.34 | 1.45 | 1.37 | 1.37 |
| APTT, sec | 38.8 | 82.6 | 60 | 45.9 | 40.3 | 40.2 |
| FVII, % | 57.5 | 21.6 | 32.1 | 54.8 | 50 | 59.6 |
| FIX, % | 59.4 | 29.5 | 23.4 | 58.9 | 61.2 | 58 |

The results show the good recovery in coagulant activity of Factor VII and Factor IX achieved as well as the normalisation of the value of all assays after 3 oil extractions.

### EXAMPLE 10: Viral inactivation of 150 ml cryoprecipitate with 1% TnBP and 1% Triton X-45

150 ml cryoprecipitate were treated with 1% TnBP and 1% Triton X-45 according to the protocol of Example 4.

The TnBP and Triton X-45 contents are measured in the cryoprecipitate after each extraction step:

| **Step** | **TnBP content measured by gas chromatography** | **Triton X-45 content measured by HPLC** |
|---|---|---|
| Cryoprecipitate with 1% TnBP & 1% Triton X-45 | 1% | 1% |
| Cryoprecipitate after 1 oil extraction | 570ppm | 0.4% |
| Cryoprecipitate after 2 oil extractions | < 10ppm | 730 ppm |
| Cryoprecipitate after 3 oil extractions | Undetectable (< 10 ppm) | < 10 ppm |

The results show that TnBP and Triton X-45 are below 10 ppm in the final Cryoprecipitate after 3 oil extractions.

The PT, and FVIII, fibrinogen, and von Willebrand factor and FIX physiological functions are also determined at all steps:

| | Cryoprecipitate Start | Cryoprecipitate Final |
|---|---|---|
| PT, sec | 12.73 | 14.5 |
| FVIII, iu/ml | 6.75 | 6.85 |
| Fibrinogen, g/l | 6.66 | 6.56 |
| Von Willebrand factor, RCo, iu/ml | 8.50 | 8.50 |

The results show the good recovery in Factor VIII, fibrinogen, and von Willebrand factor activity achieved after 3 oil extractions.

### EXAMPLE 11: Viral inactivation of 150 ml cryoprecipitate with 1% TnBP and 1% Triton X-100

150 ml cryoprecipitate were treated with 1% TnBP and 1% Triton X-100 according to the protocol of Example 5.

The TnBP and Triton X-100 contents are measured in the cryoprecipitate after each extraction step and after column chromatography:

| **Step** | **TnBP content measured by gas chromatography** | **Triton X-100 content measured by HPLC** |
|---|---|---|
| cryoprecipitate with 1% TnBP & 1% Triton X-100 | 1% | 1% |
| cryoprecipitate after 1 oil extraction | 450 ppm | 0.92% |
| cryoprecipitate after 2 oil extractions | 85 ppm | 0.87% |
| cryoprecipitate after 3 oil extractions | Undetectable (< 10 ppm) | 0.85% |
| cryoprecipitate after column chromatography | Undetectable (< 10 ppm) | < 10 ppm |

The results show that TnBP and Triton X-100 are not detectable (< 10 ppm) in the final cryoprecipitate after 3 oil extractions and column chromatography, respectively.

The FVIII, fibrinogen, and VWF physiological functions are also determined at all steps:

| | Plasma | SD | 1 oil | 2 oil | 3 oil | Chromato graphy |
|---|---|---|---|---|---|---|
| FVIII, iu/ml | 8.5 | 7.2 | 7.8 | 7.6 | 9.3 | 9.1 |
| Fibrinogen,mg/ml | 10.5 | 10.2 | 10.5 | 11 | 11 | 10.7 |
| VWFRCoiu/ml | 10.2 | 10.1 | 11.5 | 9.8 | 9.8 | 10.2 |

The results show the good recovery in coagulant activity achieved after 3 oil extractions.

### EXAMPLE 12 : Viral inactivation of 150 ml cryoprecipitate with 2% TnBP at 37 °C

150 ml cryoprecipitate were treated with 2% TnBP according to the protocol of Example 6.

The TnBP is measured in the cryoprecipitate after each extraction step:

| **Step** | **TnBP content measured by Gas chromatography** |
|---|---|
| cryoprecipitate with 2% TnBP | 2% |
| cryoprecipitate after 1 oil extraction | 420 ppm |
| cryoprecipitate after 2 oil extractions | 101 ppm |
| cryoprecipitate after 3 oil extractions | Undetectable (< 10 ppm) |

The results show that TnBP is not detectable (< 10 ppm) in the final cryoprecipitate after 3 oil extractions.

The FVIII, fibrinogen, and VWF physiological functions are also determined at all steps :

| | Plasma | SD | 1 oil | 2 oil | 3 oil |
|---|---|---|---|---|---|
| FVIII, iu/ml | 6.5 | 5.8 | 5.9 | 6.0 | 6.7 |
| VWFRCo, iu/ml | 8.6 | 7.6 | 8.2 | 8.4 | 8.6 |
| Fibrinogen, mg/ml | 9.2 | 7.7 | 9.0 | 9.2 | 9.3 |

The results show the good recovery in coagulant activity achievedafter 3 oil extractions.

## Claims

1. Set system of disposable bags comprising at least one viral inactivation bag (1), comprising an inner compartment (4), an inlet facility (5) and an outlet facility (6), which are both connected to the inner compartment (4), wherein the inner compartment (4) of the the bag (1) has an ovoid longitudinal section and the inlet facility (5) and the outlet facility (6) of the viral inactivation bag (1) are arranged on opposite sides of the bag (1), and at least one funnel bag (9), said funnel bag (9) comprises an inner compartment (10), an inlet facility (11) and an outlet facility (12), which are both connected to the inner compartment (10), the inner compartment (10) having a lower portion in the form of a funnel that meets the outlet facility (12) and the inner compartment (10) holds pharmaceutical grade oil, the different bags being connectable with each other.

2. The set system of disposable bags according to claim 1, **characterised in that** it comprises two viral inactivation bags (1).

3. The set system of disposable bags according to claim 1 or 2, **characterised in that** the cross section of the inner compartment (4) is elliptic.

4. The set system of disposable bags according claim 3, **characterised in that** the inner compartment (4) of the viral inactivation bag (1) has an elliptical longitudinal section with a first axis (a) and a second axis (b), the first axis (a) being parallel to the horizontal sides (2) of the bag (1) and the second axis (b) being parallel to the vertical sides (3) of the bag (1) and the ratio a/b being greater than 1.

5. The set system of disposable bags according to any of claims 1 to 4, **characterised in that** the volume of the inner compartment (4) is in the range from 50 ml to 20000 ml, preferably in the range from 50 ml to 5000 ml, still preferably from 100 ml to 3000 ml and even more preferably from 200 ml to 2000 ml.

6. The set system of disposable bags according to any of claims 1 to 5, **characterised in that** the bags (1, 9, 15) are made of medical/pharmaceutical grade polyvinylchloride.

7. The set system of disposable bags according to claims 1 to 6, **characterised in that** the bags are connected in such a way to ensure sterility of the biological fluid to be treated.

8. The set system of disposable bags according to claims 1 to 6, **characterised in that** the pharmaceutical grade oil is sterile.

9. Method of viral inactivation of a biological fluid comprising the steps of
a.viral inactivation of the biological fluid in a viral inactivation bag using the S/D (solvent/detergent) method, the solvent only method or the detergent only method, wherein the process chemicals that are added to the biological fluid are solvent/detergent combinations, solvent or combinations of solvents alone or detergent or combinations of detergents alone, said viral inactivation bag comprising an inner compartment (4), an inlet facility (5) and an outlet facility (6), which are both connected to the inner compartment (4), wherein the inner compartment (4) of the bag (1) has an ovoid longitudinal section and the inlet facility (5) and the outlet facility (6) of the viral inactivation bag (1) are arranged on opposite sides of the bag (1)
b. oil extraction of the solvent and/or detergent from the biological fluid in a pharmaceutical grade oil containing funnel bag, said funnel bag (9) comprises an inner compartment (10), an inlet facility (11) and an outlet facility (12), which are both connected to the inner compartment (10), the inner compartment (10) having a lower portion in the form of a funnel that meets the outlet facility (12).

10. Method of viral inactivation according to claim 9, **characterised in that** the biological fluid is selected from the group comprising mammalian blood, blood plasma, blood serum, plasma fractions, precipitates from blood fractions and supernatants from blood fractionation, platelet poor plasma, cryo-poor plasma.

11. Method of viral inactivation according to claim 9 or 10, **characterised in that** the viral inactivation method is the S/D (solvent/detergent) method or the solvent only method.

12. Method of viral inactivation according to claim 11, **characterised in that** the solvent is selected from the group of tri-(n-butyl)phosphate, tri-(t-butyl)phosphate, tri-(n-hexyl)phosphate, tri-(2-ethylhexyl) phosphate, tri-(n-decyl)phosphate, di-(n-butyl)phosphate, di-(t-butyl)phosphate, di-(n-hexyl)phosphate, di-(2-ethylhexyl)phosphate, di-(n-decyl)phosphate, ethyl di(n-butyl) phosphate or mixtures thereof.

13. Method of viral inactivation according to claim 11 or 12, **characterised in that** the viral inactivation method is the solvent/detergent method and the detergent is selected from the group comprising polyoxyethylene derivatives of fatty acids, partial esters of sorbitol anhydrides, non-ionic oil soluble water detergents, sodium deoxycholate and sulfobetaines or mixtures thereof.

14. Method of viral inactivation according to claim 11 or 12, **characterised in that** step a is carried out during a period of 4 to 8 hours, preferably 4 to 6 hours, preferably about 4 hours.

15. Method of viral inactivation according to any of claims 11 to 13, **characterised in that** step b is repeated 1 to 3 times.

16. Method of viral inactivation according to any of claims 11 to 14, **characterised in that** the pharmaceutical grade oil in step b is selected from the group consisting of castor oil, soybean oil, sunflower oil, cottonseed oil, triolein, tristearin, tripalmitin, trimyristin, and combinations thereof.

17. Method of viral inactivation according to any of claims 11 to 16, **characterised in that** the pharmaceutical grade oil in step b is used in an amount from 2-20 weight% based on the weight of the biological fluid, preferably from 5 to 15 weight% and more preferably from 5 to 10 weight%.

18. Method of viral inactivation according to any of claims 11 to 16, **characterised in that** the biological fluid is selected from the group consisting of blood plasma, cryo-poor plasma and cryoprecipitate, the viral inactivation method is the solvent only method, the solvent is tri-(n-butyl)phosphate, and step b is carried out 3 times.

19. Method of viral inactivation according to any of claims 11 to 16, **characterised in that** the biological fluid is selected from the group consisting of blood plasma, cryo-poor plasma and cryoprecipitate, the viral inactivation method is the S/D method, the solvent is tri-(n-butyl)phosphate, the detergent is 4-(1,1,3,3-Tetramethylbutyl)phenyl-polyethylene glycol (t-OCt-C₆H₄-(OCH₂CH₂)ₓOH, x≈ 5), and step b is carried out 3 times.

20. Method of viral inactivation according to any of claims 11 to 16, **characterised in that** the biological fluid is selected from the group consisting of blood plasma, cryo-poor plasma and cryoprecipitate, the viral inactivation method is the S/D method, the solvent is tri-(n-butyl)phosphate, the detergent is polyoxyethylene (9-10) p-t-octyl phenol (t-Oct-C₆H₄-(OCH₂CH₂)ₓOH, x= 9-10), step b is carried out 3 times and step c is carried after step b.

21. Method of viral inactivation according to any of claims 11 to 20, **characterised in that** it further comprises a step c of chromatographic separation of the biological fluid.

22. Use of the set system of disposable bags according to any of claims 1 to 8 for the viral inactivation of biological fluids.

## Patentansprüche

1. Setsystem von Wegwerfbeuteln umfassend mindestens einen viralen Inaktivierungsbeutel (1), umfassend eine innere Kammer (4), eine Eingangsvorrichtung (5) und eine Ausgangsvorrichtung (6), welche beide mit der inneren Kammer (4) verbunden sind, wobei die innere Kammer (4) von dem Beutel (1) einen ovalen Längsschnitt aufweist und die Eingangsvorrichtung (5) und die Ausgangsvorrichtung (6) von dem viralen Inaktivierungsbeutel (1) an gegenüberliegenden Seiten des Beutels (1) angeordnet sind, und mindestens einen Trichterbeutel (9), wobei der Trichterbeutel (9) eine innere Kammer (10), eine Eingangseinrichtung (11) und eine Ausgangseinrichtung (12) umfasst, welche beide mit der inneren Kammer (10) verbunden sind, wobei die innere Kammer (10) einen unteren Abschnitt in Form eines Trichters aufweist, der mit der Ausgangseinrichtung (12) zusammen trifft und die innere Kammer (10) Öl von pharmazeutischer Qualität enthält, wobei die verschiedenen Beutel miteinander verbunden werden können.

2. Setsystem von Wegwerfbeuteln nach Anspruch 1, **dadurch gekennzeichnet, dass** es zwei virale Inaktivierungsbeutel (1) umfasst.

3. Setsystem von Wegwerfbeuteln nach Anspruch 1 oder 2, **dadurch gekennzeichnet, dass** der Querschnitt von der inneren Kammer (4) elliptisch ist.

4. Setsystem von Wegwerfbeuteln nach Anspruch 3, **dadurch gekennzeichnet, dass** die innere Kammer (4) des viralen Inaktivierungsbeutels (1) einen elliptischen Längsschnitt mit einer ersten Achse (a) und einer zweiten Achse (b) aufweist, wobei die erste Achse (a) parallel zu den horizontalen Seiten (2) von dem Beutel (1) ist und die zweite Achse (b) parallel zu den vertikalen Seiten (3) von dem Beutel (1) ist und das Verhältnis a/b größer als 1 ist.

5. Setsystem von Wegwerfbeuteln nach einem der Ansprüche 1 bis 4, **dadurch gekennzeichnet, dass** das Volumen der inneren Kammer (4) in einem Bereich von 50 ml bis 20000 ml ist, bevorzugt in einem Bereich von 50 ml bis 5000 ml, noch bevorzugter von 100 ml bis 3000 ml und noch mehr bevorzugt von 200 ml bis 2000 ml.

6. Setsystem von Wegwerfbeuteln nach einem der Ansprüche 1 bis 5, **dadurch gekennzeichnet, dass** die Beutel (1, 9, 15) aus Polyvinylchlorid von medizinischer/pharmazeutischer Qualität gemacht sind.

7. Setsystem von Wegwerfbeuteln nach Ansprüchen 1 bis 6, **dadurch gekennzeichnet, dass** die Beutel so miteinander verbunden sind, dass Sterilität der zu behandelnden biologischen Flüssigkeit gewährleiset ist.

8. Setsystem von Wegwerfbeuteln nach Ansprüchen 1 bis 6, **dadurch gekennzeichnet, dass** das Öl von pharmazeutischer Qualität steril ist.

9. Verfahren von viraler Inaktivierung von einer biologischen Flüssigkeit umfassend die Schritte von
a. viraler Inaktivierung der biologischen Flüssigkeit in einem viralen Inaktivierungsbeutel unter Verwendung des S/D (Lösungsmittel/Detergenz (solvent/detergent))-Verfahrens, des Nur-Lösungsmittel-Verfahrens (solvent only method) oder des Nur-Detergenz-Verfahrens (detergent only method), wobei die Prozesschemikalien, welche der biologischen Flüssigkeit zugesetzt werden, Lösungsmittel-/Detergenzkombinationen, Lösungsmittel oder Kombinationen von Lösungsmitteln alleine oder Detergenz oder Kombinationen von Detergenzien alleine sind, wobei der virale Inaktivierungsbeutel eine innere Kammer (4), eine Eingangsvorrichtung (5) und eine Ausgangsvorrichtung (6) aufweist, welche beide mit der inneren Kammer (4) verbunden sind, wobei die innere Kammer (4) von dem Beutel (1) einen ovalen Längsschnitt aufweist und die Eingangsvorrichtung (5) und die Ausgangsvorrichtung (6) von dem viralen Inaktivierungsbeutel (1) an gegenüberliegenden Seiten des Beutels (1) angeordnet sind
b. Ölextraktion von dem Lösungsmittel und/oder Detergenz von der biologischen Flüssigkeit in einem Trichterbeutel, welcher Öl pharmazeutischer Qualität enthält, wobei der Trichterbeutel (9) eine innere Kammer (10), eine Eingangseinrichtung (11) und eine Ausgangseinrichtung (12) umfasst, welche beide mit der inneren Kammer (10) verbunden sind, wobei die innere Kammer (10) einen unteren Abschnitt in Form eines Trichters aufweist, der mit der Ausgangseinrichtung (12) zusammen trifft.

10. Verfahren von viraler Inaktivierung nach Anspruch 9, **dadurch gekennzeichnet, dass** die biologische Flüssigkeit ausgewählt ist aus einer Gruppe umfassend Säugetierblut, Blutplasma, Blutserum, Plasmafraktionen, Niederschläge von Blutfraktionen und Überstände von Blutfraktionierung, plättchenarmes Plasma, cryoarmes Plasma.

11. Verfahren von viraler Inaktivierung nach Anspruch 9 oder 10, **dadurch gekennzeichnet, dass** das virale Inaktivierungsverfahren das S/D (Lösungsmittel/Detergenz)-Verfahren oder das Nur-Lösungsmittel-Verfahren (solvent only method) ist.

12. Verfahren von viraler Inaktivierung nach Anspruch 11, **dadurch gekennzeichnet, dass** das Lösungsmittel ausgewählt ist aus der Gruppe von Tri-(n-butyl)phosphat, Tri-(t-butyl)phosphat, Tri-(n-hexyl)phosphat, Tri-(2-ethylhexyl)phosphat, Tri-(n-decyl)phosphat, Di-(n-butyl)phosphat, Di-(t-butyl)phosphat, Di-(n-hexyl)phosphat, Di-(2-ethylhexyl)phosphat, Di-(n-decyl)phosphat, Ethyl-di(n-butyl)phosphat oder Mischungen davon.

13. Verfahren von viraler Inaktivierung nach Anspruch 11 oder 12, **dadurch gekennzeichnet, dass** das virale Inaktivierungsverfahren das Lösungsmittel/Detergenzverfahren ist und das Lösungsmittel ausgewählt ist aus der Gruppe umfassend Polyoxyethylenderivate von Fettsäuren, partiellen Estern von Sorbitolanhydriden, nicht-ionischen öllöslichen Wasserdetergenzien, Natriumdeoxycholat und Sulfobetainen oder Mischungen davon.

14. Verfahren von viraler Inaktivierung nach Anspruch 11 oder 12, **dadurch gekennzeichnet, dass** Schritt a während eines Zeitraums von 4 bis 8 Stunden, bevorzugt 4 bis 6 Stunden, bevorzugt etwa 4 Stunden durchgeführt wird.

15. Verfahren von viraler Inaktivierung nach einem der Ansprüche 11 bis 13, **dadurch gekennzeichnet, dass** Schritt b 1- bis 3-mal wiederholt wird.

16. Verfahren von viraler Inaktivierung nach einem der Ansprüche 11 bis 14, **dadurch gekennzeichnet, dass** das Öl von pharmazeutischer Qualität in Schritt b ausgewählt ist aus der Gruppe bestehend aus Rizinusöl, Sojabohnenöl, Sonnenblumenöl, Baumwollsamenöl, Triolein, Tristearin, Tripalmitin, Trimyristin und Kombinationen davon.

17. Verfahren von viraler Inaktivierung nach einem der Ansprüche 11 bis 16, **dadurch gekennzeichnet, dass** das Öl von pharmazeutischer Qualität in Schritt b in einer Menge von 2-20 Gewichts-% basierend auf dem Gewicht der biologischen Flüssigkeit, bevorzugt von 5 bis 15 Gewichts-% und mehr bevorzugt von 5 bis 10 Gewichts-% verwendet wird.

18. Verfahren von viraler Inaktivierung nach einem der Ansprüche 11 bis 16, **dadurch gekennzeichnet, dass** die biologische Flüssigkeit ausgewählt ist aus der Gruppe bestehend aus Blutplasma, cryoarmem Plasma und Cryoniederschlag, wobei das virale Inaktivierungsverfahren das Nur-Lösungsmittel-Verfahren (solvent only method) ist, wobei das Lösungsmittel Tri-(n-butyl)phosphat ist und Schritt b 3-mal durchgeführt wird.

19. Verfahren von viraler Inaktivierung nach einem der Ansprüche 11 bis 16, **dadurch gekennzeichnet, dass** die biologische Flüssigkeit ausgewählt ist aus der Gruppe bestehend aus Blutplasma, cryoarmem Plasma und Cryoniederschlag, wobei das virale Inaktivierungsverfahren das S/D-Verfahren ist, wobei das Lösungsmittel Tri-(n-butyl)phosphat ist, das Detergenz 4-(1,1,3,3-Tetramethylbutyl)phenyl-polyethylenglycol (t-Oct-C₆H₄-(OCH₂CH₂)ₓOH, x≈ 5) ist und Schritt b dreimal durchgeführt wird.

20. Verfahren von viraler Inaktivierung nach einem der Ansprüche 11 bis 16, **dadurch gekennzeichnet, dass** die biologische Flüssigkeit ausgewählt ist aus der Gruppe bestehend aus Blutplasma, cryoarmem Plasma und Cryoniederschlag, wobei das virale Inaktivierungsverfahren das S/D-Verfahren ist, das Lösungsmittel Tri-(n-butyl)phosphat ist, das Detergenz Polyoxyethylen (9-10) p-t-Octylphenol (t-Oct-C₆H₄-(OCH₂CH₂)ₓOH, x = 9-10) ist, Schritt b dreimal durchgeführt wird und Schritt c nach Schritt b ausgeführt wird.

21. Verfahren von viraler Inaktivierung nach einem der Ansprüche 11 bis 20, **dadurch gekennzeichnet, dass** es weiterhin einen Schritt c von chromatographischer Trennung der biologischen Flüssigkeit umfasst.

22. Verwendung des Setsystems von Wegwerfbeuteln nach Ansprüchen 1 bis 8 zur viralen Inaktivierung von biologischen Flüssigkeiten.

## Revendications

1. Système de sacs jetables comprenant au moins un sac d'inactivation virale (1), comprenant un compartiment intérieur (4), un moyen d'entrée (5) et un moyen de sortie (6), qui sont tous les deux reliés au compartiment intérieur (4), dans lequel le compartiment intérieur (4) du sac (1) a une section longitudinale ovoïde et le moyen d'entrée (5) et le moyen de sortie (6) du sac d'inactivation virale (1) sont disposés sur des côtés opposés du sac (1), et au moins un sac en forme d'entonnoir (9), ledit sac en forme d'entonnoir (9) comprend un compartiment intérieur (10), un moyen d'entrée (11) et un moyen de sortie (12), qui sont tous les deux reliés au compartiment intérieur (10), le compartiment intérieur (10) ayant une partie inférieure en forme d'entonnoir qui rencontre le moyen de sortie (12) et le compartiment intérieur (10) contient une huile de qualité pharmaceutique, les différents sacs pouvant être reliés les uns aux autres.

2. Système de sacs jetables selon la revendication 1, **caractérisé en ce qu'**il comprend deux sacs d'inactivation virale (1).

3. Système de sacs jetables selon la revendication 1 ou 2, **caractérisé en ce que** la section transversale du compartiment intérieur (4) est elliptique.

4. Système de sacs jetables selon la revendication 3, **caractérisé en ce que** le compartiment intérieur (4) du sac d'inactivation virale (1) a une section longitudinale elliptique avec un premier axe (a) et un second axe (b), le premier axe (a) étant parallèle aux côtés horizontaux (2) du sac (1) et le second axe (b) étant parallèle aux côtés verticaux (3) du sac (1) et le rapport a/b étant supérieur à 1.

5. Système de sacs jetables selon l'une quelconque des revendications 1 à 4, **caractérisé en ce que** le volume du compartiment intérieur (4) se trouve dans la plage de 50 mL à 20 000 mL, de préférence dans la plage de 50 mL à 5 000 mL, de manière davantage préférée de 100 mL à 3 000 mL et de manière encore davantage préférée de 200 mL à 2 000 mL.

6. Système de sacs jetables selon l'une quelconque des revendications 1 à 5, **caractérisé en ce que** les sacs (1, 9, 15) sont en chlorure de polyvinyle de qualité médicale/pharmaceutique.

7. Système de sacs jetables selon les revendications 1 à 6, **caractérisé en ce que** les sacs sont connectés de manière à assurer la stérilité du fluide biologique à traiter.

8. Système de sacs jetables selon les revendications 1 à 6, **caractérisé en ce que** l'huile de qualité pharmaceutique est stérile.

9. Procédé d'inactivation virale d'un fluide biologique qui comprend les étapes suivantes :
a. inactivation virale du fluide biologique dans un sac d'inactivation virale en utilisant le procédé S/D (solvant/détergent), le procédé n'utilisant que du solvant ou le procédé n'utilisant que du détergent, dans lequel les produits chimiques du procédé qui sont ajoutés au fluide biologique sont des combinaisons de solvant/détergent, du solvant ou des combinaisons de solvants seuls, ou du détergent ou des combinaisons de détergents seuls, ledit sac d'inactivation virale comprenant un compartiment intérieur (4), un moyen d'entrée (5) et un moyen de sortie (6), qui sont tous les deux reliés au compartiment intérieur (4), dans lequel le compartiment intérieur (4) du sac (1) a une section longitudinale ovoïde et le mécanisme d'entrée (5) et le mécanisme de sortie (6) du sac d'inactivation virale (1) sont disposés sur des côtés opposés du sac (1)
b. extraction à l'huile du solvant et/ou du détergent du fluide biologique dans un sac en forme d'entonnoir contenant une huile de qualité pharmaceutique, ledit sac en forme d'entonnoir (9) comprend un compartiment intérieur (10), un moyen d'entrée (11) et un moyen de sortie (12), qui sont tous les deux reliés au compartiment intérieur (10), le compartiment intérieur (10) ayant une partie inférieure sous la forme d'un entonnoir qui rencontre le moyen de sortie (12).

10. Procédé d'inactivation virale selon la revendication 9, **caractérisé en ce que** le fluide biologique est choisi dans le groupe comprenant du sang de mammifère, du plasma sanguin, du sérum sanguin, des fractions plasmatiques, des précipités de fractions plasmatiques et des surnageants de fractionnement sanguin, du plasma pauvre en plaquettes, du plasma pauvre en cryoprécipité.

11. Procédé d'inactivation virale selon la revendication 9 ou 10, **caractérisé en ce que** le procédé d'inactivation virale est le procédé S/D (solvant/détergent) ou le procédé n'utilisant que le solvant.

12. Procédé d'inactivation virale selon la revendication 11, **caractérisé en ce que** le solvant est choisi dans le groupe du tri-(n-butyl)phosphate, du tri-(t-butyl)phosphate, du tri-(n-hexyl)phosphate, du tri-(2-éthylhexyl)phosphate, du tri-(n-décyl)phosphate, du di-(n-butyl)phosphate, du di-(t-butyl)phosphate, du di-(n-hexyl)phosphate, du di-(2-éthylhexyl)phosphate, du di-(n-décyl)phosphate, de l'éthyl di(n-butyl) phosphate ou de leurs mélanges.

13. Procédé d'inactivation virale selon la revendication 11 ou 12, **caractérisé en ce que** le procédé d'inactivation virale est le procédé solvant/détergent et **en ce que** le détergent est choisi dans le groupe comprenant les dérivés de polyoxyéthylène d'acides gras, les esters partiels d'anhydrides de sorbitol, les détergents aqueux, liposolubles et non ioniques, le désoxycholate de sodium et les sulfobétaïnes ou leurs mélanges.

14. Procédé d'inactivation virale selon la revendication 11 ou 12, **caractérisé en ce que** l'étape a est réalisée pendant une période de 4 à 8 heures, de préférence de 4 à 6 heures, de préférence d'environ 4 heures.

15. Procédé d'inactivation virale selon l'une quelconque des revendications 11 à 13, **caractérisé en ce que** l'étape b est répétée 1 à 3 fois.

16. Procédé d'inactivation virale selon l'une quelconque des revendications 11 à 14, **caractérisé en ce que** l'huile de qualité pharmaceutique de l'étape b est choisie dans le groupe constitué de l'huile de ricin, de l'huile de soja, de l'huile de tournesol, de l'huile de coton, de la trioléine, de la tristéarine, de la tripalmitine, de la trimyristine, et de leurs combinaisons.

17. Procédé d'inactivation virale selon l'une quelconque des revendications 11 à 16, **caractérisé en ce que** l'huile de qualité pharmaceutique dans l'étape b est utilisée en une quantité de 2 à 20 % en poids en se basant sur le poids du fluide biologique, de préférence de 5 à 15 % en poids et de manière davantage préférée de 5 à 10 % en poids.

18. Procédé d'inactivation virale selon l'une quelconque des revendications 11 à 16, **caractérisé en ce que** le fluide biologique est choisi dans le groupe constitué du plasma sanguin, du plasma pauvre en cryoprécipité et du cryoprécipité, le procédé d'inactivation virale est le procédé n'utilisant que le solvant, le solvant est le tri-(n-butyl)phosphate, et l'étape b est réalisée 3 fois.

19. Procédé d'inactivation virale selon l'une quelconque des revendications 11 à 16, **caractérisé en ce que** le fluide biologique est choisi dans le groupe constitué du plasma sanguin, du plasma pauvre en cryoprécipité et du cryoprécipité, le procédé d'inactivation virale est le procédé S/D, le solvant est le tri-(n-butyl)phosphate, le détergent est le 4-(1,1,3,3-tétraméthylbutyl)phényl-polyéthylène glycol (t-Oct-C₆H₄-(OCH₂CH₂)OH, x≈ 5), et l'étape b est réalisée 3 fois.

20. Procédé d'inactivation virale selon l'une quelconque des revendications 11 à 16, **caractérisé en ce que** le fluide biologique est choisi dans le groupe constitué du plasma sanguin, du plasma pauvre en cryoprécipité et du cryoprécipité, le procédé d'inactivation virale est le procédé S/D, le solvant est le tri-(n-butyl)phosphate, le détergent est le polyoxyéthylène (9-10) p-t-octyl phénol (t-Oct-C₆H₄-(OCH₂CH₂)ₓOH, x = 9-10), l'étape b est réalisée 3 fois et l'étape c est réalisée après l'étape b.

21. Procédé d'inactivation virale selon l'une quelconque des revendications 11 à 20, **caractérisé en ce qu'**il comprend en outre une étape c de séparation chromatographique du fluide biologique.

22. Utilisation du système de sacs jetables selon l'une quelconque des revendications 1 à 8, pour l'inactivation virale de fluides biologiques.
